# EUROPEAN PATENT APPLICATION

(11) **EP 1 967 378 A1**
(43) Date of publication of application: **10.09.2008**
(21) Application number: 06843413.3
(22) Date of filing: 27.12.2006
(51) Int. Cl.: B41M 5/26, C07D 209/14, C07D 405/12, C09B 23/00, G11B 7/24, G11B 7/244

(54) **OPTICAL RECORDING MEDIUM AND AZACYANINE DYE**

(30) Priority: 27.12.2005 JP 2005375871; 23.06.2006 JP 2006174427
(71) Applicant: Mitsubishi Kagaku Media Co., Ltd., Tokyo 108-8415 (JP)
(72) Inventor: KUROSE, Yutaka, Tokyo 108-0014 (JP); MIYAZAWA, Takashi, Tokyo 108-0014 (JP); KUBO, Hideyuki, Tokyo 180-0014 (JP); UCHIDA, Naoyuki, Tokyo 180-0014 (JP); FUROMOTO, Shigeyuki, Tokyo 180-0014 (JP); SATAKE, Kenichi, Tokyo 180-0014 (JP); SHODA, Hisashi, Tokyo 180-0014 (JP); AIZAWA, Yasushi, Okayama-shi Okayama 700-0907 (JP); DAN-OH, Yasufumi, Okayama-shi Okayama 700-0907 (JP); TOKI, Masahiko, Okayama-shi Okayama 700-0907 (JP)
(74) Representative: HOFFMANN EITLE
(86) International application number: PCT/JP2006/326031
(87) International publication number: WO 2007/074861

(57) **Abstract**

An optical recording medium on which high-density recording and reading of optical information can be conducted by a short-wavelength light such as a blue laser beam is provided.

In an optical recording medium comprising at least a substrate and a recording layer thereon that can record or read information by irradiation with light, the recording layer contains an azacyanine dye represented by general formula [I]: 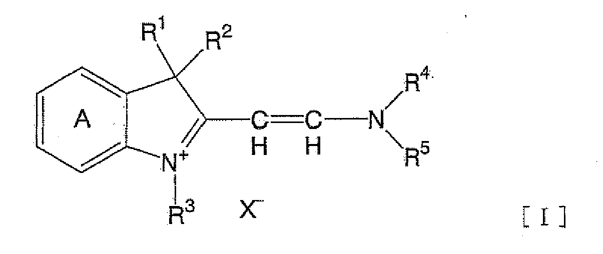 wherein R¹ and R² each independently represent a hydrogen atom or an optionally substituted linear or branched alkyl group having one to four carbon atoms; R³ represents a hydrogen atom or a hydrocarbon group; R⁴ represents a hydrogen atom or a linear or branched alkyl group having one to four carbon atoms; R⁵ represents an optionally substituted aromatic ring group or an optionally substituted unsaturated heterocyclic group; R⁴ and R⁵ may be combined together to form a ring; X⁻ represents a counter anion; and the benzene ring A may be optionally substituted.

## Description

### Technical Field

The present invention relates to optical recording media and azacyanine dyes. More particularly, it relates to optical recording media that are applicable to blue lasers and have excellent light resistance, and azacyanine dyes suitable for use in the recording layers of the optical recording media.

### Background Art

At present, various optical recording media such as CD-R/RWs (compact disc-recordable/rewritable), DVD-R/RWs (digital video disc-recordable/rewritable), and MO (magnetooptic) discs are widely known and used as external storage devices for information processors such as computers, due to their large capacity of information storage and ease of random access. Among these media, organic-dye optical recording media represented by CD-R or DVD-R, each provided with a recording layer containing an organic dye compound are believed to be superior in that they can be easily produced at low costs.

As the amount of information processed increases, the media with higher recording density are required. In recent years, optical recording media have been proposed on which high density recording/reading can be conducted by a laser beam with a short oscillation wavelength, such as a blue laser beam that has been remarkably improved.

For such optical recording media using a laser beam with a short oscillation wavelength, such as a blue laser beam, some examples have been reported. However, no unified standard as yet are established for recording devices or recording conditions. It is therefore actually difficult to envisage desired optical recording media.

In such circumstance, for example, the following Patent Documents 1 to 4 each disclose an organic dye for the recording layer of an optical recording medium on which information can be recorded and read by a laser beam with a short oscillation wavelength of approximately 405 to 430 nm.

[Patent Document 1] Japanese Unexamined Patent Application Publication No. HEI 11-105423
[Patent Document 2] Japanese Unexamined Patent Application Publication No. HEI 11-78239
[Patent Document 3] International Publication No. W02006/035554
[Patent Document 4] Japanese Unexamined Patent Application Publication No. 2001-301333

### Disclosure of Invention

### Problems to be solved by the Invention

However, conventional organic dyes for short wavelengths such as a blue laser beam as described in Patent Document 1 or 2 have the following problems of little absorption of semiconductor laser beams currently developed, i.e. laser beams in the vicinity of a wavelength of 405 nm, or low solubility in organic solvents. In particular, conventional organic dyes for short wavelengths have structural hurdles of difficult adjustment of the wavelength to be absorbed. Accordingly, optical recording media with the recording layers containing those organic dyes disadvantageously have poor recording sensitivity.

Organic dyes for short wavelengths such as blue laser as described in Patent Document 3 or 4 are directed to the recording mechanism similar to that of conventional optical recording media called CD-R or DVD-R, i.e., High-to-Low recording that is a recording method in which reflectivity during non-recording is higher than that during recording. For this recording, a recording material producing much return light from the unrecorded part is considered to be excellent. However, a recording method using blue semiconductor lasers currently developed, for example, HD (High Definition) DVD-Rs or BD (Blu-ray Disc)-Rs has a disadvantage of being difficult to put the High-to-Low recording into practice.

The present invention has been accomplished in consideration of the problems above. Objects of the present invention are to provide an optical recording medium on which high-density recording and reading of optical information can be conducted by short-wavelength light beams such as a blue laser beam, and a novel dye suitably used in this optical recording medium.
Another object of the present invention is to provide an optical recording medium on which high density optical information recording and reading can be conducted by Low-to-High recording that is a recording mechanism different from the conventional one, and a novel dye suitably used in this optical recording medium. Low-to-High recording is a recording method in which reflectivity during recording is higher than that during non-recording. For this recording method, it is desired to decrease reflectivity during non-recording.

### Means for solving the Problems

As a result of extensive studies, the inventors have discovered the following fact and accomplished the present invention: Use of an azacyanine dye of a particular structure in a recording layer of an optical recording medium enables high-density recording and reading of optical information by short-wavelength light beams such as a blue laser beam, and particularly can put this medium as a novel optical recording medium by Low-to-High recording into practice.

That is, an aspect of present invention provides an optical recording medium comprising at least a substrate and a recording layer thereon that can record or read information by irradiation with light, the recording layer containing an azacyanine dye represented by the following general formula [I] (claim 1): 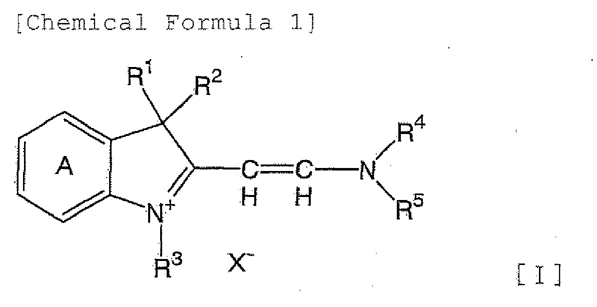 wherein R¹ and R² each independently represent a hydrogen atom or an optionally substituted linear or branched alkyl group having one to four carbon atoms; or R¹ and R² may be combined together to form a ring;
R³ represents a hydrogen atom or a hydrocarbon group, and R³ may crosslink with two or more compounds of general formula [I] when R³ represents a hydrocarbon group;
R⁴ represents a hydrogen atom or a linear or branched alkyl group having one to four carbon atoms; and
R⁵ represents an optionally substituted aromatic ring group or an optionally substituted unsaturated heterocyclic group, and R³ may combine with two or more compounds of general formula [I] ;
provided that R⁴ and R⁵ may be combined together to form a ring;
X⁻ represents a counter anion; and
the benzene ring A may be optionally substituted.

Preferably, in above-described general formula [I], R⁴ represents a hydrogen atom, and R⁵ represents an optionally substituted phenyl group (claim 2).

Also preferably, in above-described general formula [I], R⁴ represents a hydrogen atom, and R⁵ represents an optionally substituted five- or six-membered unsaturated heterocyclic group (claim 3).

Also preferably, R⁴ and R⁵ in above-described general formula [I] are combined to form a five- or six-membered saturated hydrocarbon group or saturated heterocycle (claim 4).

Preferably, R⁵ represents an unsubstituted phenyl group (claim 5).

Preferably, X⁻ in general formula [I] is an azo-metal complex anion represented by the following general formula [II] (claim 6): 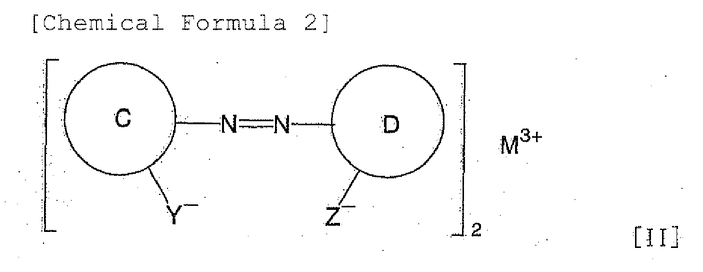 wherein these rings C and D each independently represent an aromatic ring or a heterocycle provided that at least one of these rings C and D is a heterocycle; Y and Z each independently represent a group having active hydrogen; and M represents a trivalent metal element.

Preferably, on the recording layer, recording or reading of information is conducted using a laser beam with a wavelength of 350 nm to 530 nm (claim 7).

Another aspect of the present invention provides an azacyanine dye represented by the following formula (i) (claim 8) : 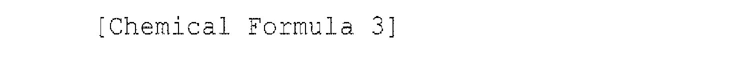 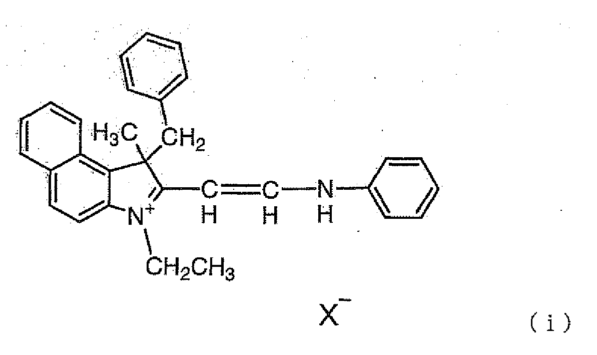 wherein X⁻ represents a counter anion.

### Advantages

Use of the optical recording media of the present invention and the azacyanine dye of the present invention enables high-density recording and reading of optical information by short-wavelength light beams such as a blue laser beam, and also by Low-to-High recording having a recording mechanism different from the conventional one.

### Brief Description of the Drawings

[Fig. 1] Fig. 1(a) is a partial cross-sectional view that schematically illustrates an example of a layered structure of an optical recording medium according to a first embodiment of the present invention, and Fig. 1(b) is a partial cross-sectional view that schematically illustrates an example of a layered structure of an optical recording medium according to a second embodiment of the present invention.
[Fig. 2] Fig. 2 is an absorption spectrum from a coated film of an azacyanine dye (Exemplary Compound (1)) prepared in Example 1.
[Fig. 3] Fig. 3 is an absorption spectrum from a coated film of an azacyanine dye (Exemplary Compound (2)) prepared in Example 2.
[Fig. 4] Fig. 4 is an absorption spectrum from a coated film of an azacyanine dye prepared in Comparative Example 1.
[Fig. 5] Fig. 5 shows relationships between the laser recording power and PRSNR in optical recording media of Examples A to E.
[Fig. 6] Fig. 6 shows the relationship between the laser recording power and PRSNR in the optical recording medium (a dual layered medium) of Example F.
[Fig. 7] Each of (a) to (f) illustrates a process of producing a dual layered optical recording medium according to a third embodiment of the present invention.

### Description of Reference Numerals

- 1: substrate
- 2: recording layer
- 3: reflective layer
- 4: protective layer
- 5: protective coating
- 10, 20: optical recording media
- L: laser beam
- 201: first substrate
- 202: first recording layer
- 203: first reflective layer
- 204: intermediate layer
- 204a: ultraviolet-curing resin layer
- 205: second recording layer
- 206: second reflective layer
- 207: adhesive layer
- 208: second substrate (mirror substrate)
- 210: resin stamper

### Best Mode for Carrying Out the Invention

The present invention will now be described in detail with reference to embodiments, but should not be limited to the description below. Various modifications can be made within the scope of the invention.

The optical recording medium of the present invention includes a substrate and a recording layer thereon that can record or read information by irradiation with light, the recording layer containing an azacyanine dye represented by general formula [I] described below. For convenience, the azacyanine dye in a recording layer of the optical recording medium of the present invention will be first described in the description below, and subsequently the optical recording medium of the present invention will be described.

### [I. Azacyanine Dye]

The optical recording medium of the present invention contains an azacyanine dye represented by the following general formula [I] in the recording layer (hereinafter abbreviated as "azacyanine dye of formula [I]"). The azacyanine dye of formula [I] has appropriate absorption in the blue light region with a wavelength of 350 nm to 530 nm, and therefore is a dye compound which is suitable for recording by a blue laser beam and has practicable light resistance.

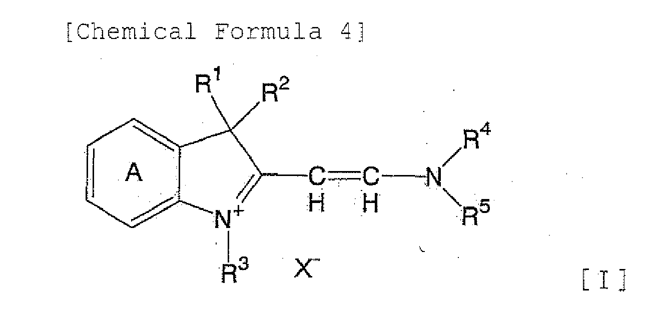

wherein R¹ and R² each independently represent a hydrogen atom or a linear or branched alkyl group having one to four carbon atoms; or R¹ and R² may be combined together to form a ring;
R³ represents a hydrogen atom or a hydrocarbon group, and R³ may crosslink with two or more compounds of general formula [I] when R³ represents a hydrocarbon group;
R⁴ represents a hydrogen atom or a linear or branched alkyl group having one to four carbon atoms;
R⁵ represents an optionally substituted aromatic ring group or an optionally substituted unsaturated heterocyclic group, and R⁵ may combine with two or more compounds of general formula [I];
provided that R⁴ and R⁵ may be combined together to form a ring;
X⁻ represents a counter anion; and
the benzene ring A may be optionally substituted.

Each of R¹ to R⁵ in general formula [I] will now be described.
The phrase "optionally substituted" used in the description of R¹ to R⁵ means that the substituents generally include no water-soluble group such as a hydroxyl group and a sulfonate group, unless otherwise stated. This is because a dye compound must have solubility in an organic solvent to a certain extent such that the dye compound can be contained in the recording layer of the optical recording medium, and because such a recording layer must be a stable film having water resistance to a certain extent for practical use of optical discs.

In general formula [I], and R² each independently represent a hydrogen atom or an optionally substituted linear or branched alkyl group having one to four carbon atoms, or R¹ and R² may also be combined together to form a ring. Examples of the linear or branched alkyl group having one to four carbon atoms include methyl group, ethyl group, propyl group, isopropyl group, butyl group, isobutyl group, sec-butyl group, and tert-butyl group.

When R¹ or R² is a linear or branched alkyl group having one to four carbon atoms, examples of the substituent for the group include saturated or unsaturated cyclic groups such as azulene ring, quinoline ring, cyclopropane ring, cyclobutane ring, cyclopentane ring, cyclohexane ring, cyclohexene ring, naphthalene ring, thiophene ring, benzene ring, piperidine ring, pyridine ring, pyrrolizine ring, pyrrole ring, and furan ring, and halogen atoms (such as fluorine atom, chlorine atom, and bromine atom). Among these substituents preferred are benzene ring and naphthalene ring, and especially preferred is an alkyl group having one or two carbon atoms bound with a benzene ring as R¹ or R².
Examples of the ring which R¹ and R² are combined together to form include saturated or unsaturated cyclic structures such as cyclobutane ring, cyclopentane ring, cyclohexane ring, cyclohexene ring, and cycloheptane ring.

When R¹ or R² has a cyclic substituent and R¹ and R² are combined together to form a ring, such a cyclic substituent or ring may have one or more substituents. Examples of the substituents include aliphatic hydrocarbon groups such as methyl group, ethyl group, propyl group, isopropyl group, isopropenyl group, 1-propenyl group, 2-propenyl group, 2-propynyl group, butyl group, isobutyl group, sec-butyl group, tert-butyl group, 2-butenyl group, 1,3-butadienyl group, pentyl group, isopentyl group, neopentyl group, tert-pentyl group, 1-methylpentyl group, 2-methylpentyl group, and 2-penten-4-inyl group, alicyclic hydrocarbon groups such as cyclopropyl group, cyclobutyl group, cyclopentyl group, cyclohexyl group, and cyclohexenyl group, aromatic hydrocarbon groups such as phenyl group, o-tolyl group, m-tolyl group, p-tolyl group, xylyl group, mesityl group, o-cumenyl group, m-cumenyl group, p-cumenyl group, and biphenylyl group, halogen groups such as fluoro group, chloro group, bromo group, and iodo group, alkoxy groups such as methoxy group and ethoxy group, alkylamino groups such as dimethylamino group and diethylamino group, and nitro group, and combinations thereof.

R³ in general formula [I] represents a hydrogen atom or a hydrocarbon group, and an optionally substituted linear or branched alkyl group having one to six carbon atoms is preferred when R³ represents a hydrocarbon group.
R³ may crosslink with two or more compounds of general formula [I] when R³ represents a hydrocarbon group. R³ includes linear or branched alkyl groups having one to six carbon atoms and alkyl groups and phenylene groups having more carbon atoms when R³ crosslinks with two or more compounds of general formula [I].

When R¹ to R³ are alkyl groups, they may have any substituents unless departing from the spirit of the present invention, including halogen atoms (such as fluorine atom, chlorine atom, and bromine atom), alkoxy groups (such as methoxy group, ethoxy group), alkylamino groups (such as dimethylamino group and diethylamino group), aryl groups (such as phenyl group and naphthyl group). This is because such halogen atoms improve affinity with halogenous solvents, polar groups such as alkoxy group and alkylamino group improve solvation with polar solvents, and aryl groups inhibit association of dye molecules by steric hindrance, any of which can be expected to improve solubility in solvents.

R⁴ in general formula [I] represents a hydrogen atom or a linear or branched alkyl group having one to four carbon atoms, and preferably a hydrogen atom in particular.

R⁵ in general formula [I] represents an optionally substituted aromatic ring group or an optionally substituted unsaturated heterocyclic group. The phrases "aromatic ring group" and "heterocyclic group" used herein mean an aromatic ring minus one hydrogen atom and a heterocycle minus one hydrogen atom respectively.

Preferably, R⁵ represents an unsaturated heterocycle group from the viewpoint of improved solubility of the resultant dye in an organic solvent.
When R⁵ represents an unsaturated heterocyclic group, it is preferably a five- or six-membered ring. This is because four or less membered ring structures are generally unstable, and because production of seven or more membered ring structures is fraught with complications.

When R⁵ represents an aromatic ring group, it is preferably a phenyl group.
The resultant dyes with an aromatic ring group or an unsaturated heterocycle group have light absorption at longer wavelengths compared to those with an alkyl group, and therefore have higher absorption at 405 nm that is oscillation wavelength of blue laser.

When R⁵ represents an unsaturated heterocyclic group, it includes any heterocyclic group containing a heteroatom such as nitrogen atom, oxygen atom, and sulfur atom. Preferably, the wavelength to be absorbed can be adjusted when R⁵ represents an unsaturated heterocyclic group represented by the following formula, for example.

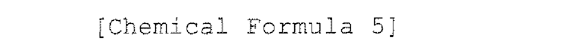

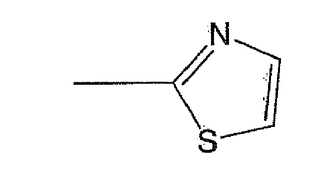

When R⁵ represents an aromatic ring group or an unsaturated heterocyclic group, such a group may have any substituent unless departing from the spirit of the present invention. Preferred examples of such substituents include linear or branched alkyl groups having one to eight carbon atoms, fluoroalkyl groups, alkoxy groups, and alkylthio groups. This is because these groups improve solubility in coating solvents, and inhibit crystallization in a thin film. Among these substituents especially preferred are linear or branched alkyl groups having one to five carbon atoms, -CF₃, -OCF₃, -SCF₃, -SC₂F₅, -OCH₃, and -O-(iso-C₃H₇).

When R⁵ represents an aromatic ring group or an unsaturated heterocyclic group, such a group may further have any fused ring unless departing from the spirit of the present invention, but preferably a five- or six-membered aromatic ring or heterocycle. Such a fused ring may have substituents described above.
R⁵ may combine with two or more compounds of general formula [I].

R⁴ and R⁵ may be combined together to form a ring. It is preferred to form such a ring, which may inhibit crystallization and improve solubility in organic solvents. The ring may have any ring structure, but preferably be a five- or six-membered ring. This is because four or less membered ring structures are generally unstable, and because production of seven or more membered ring structures is fraught with complications. Ring skeleton atoms other than nitrogen atom to which R⁴ and R⁵ are attached may be all carbon atoms, and contain a heteroatom such as nitrogen atom, oxygen atom, and sulfur atom.

When R⁴ and R⁵ taken together form a ring, the ring may have any substituents without limitation. Examples include various substituents described above as ones that an aromatic ring group or a heterocycle group R⁵ may have.

When R⁴ and R⁵ taken together form a ring, this ring structure may further have any fused ring without limitation. Examples include various fused rings described above as ones that an aromatic ring group or a heterocycle group R⁵ may have. The fused ring may also have substituents described above.
When R⁴ and R⁵ taken together form a ring, the ring structure may combine with two or more compounds of general formula [I].

The benzene ring A in general formula [I] may be optionally substituted. Such substituents include halogen atoms such as Cl and Br, linear or branched alkyl groups having one to four carbon atoms, acetylamino groups, and alkoxy groups having one to four carbon atoms. The benzene ring A may further have any fused ring, but preferably a benzene ring.

X⁻ in general formula [I] represents a counter anion. The counter anion may be any monovalent anion, including I⁻, Cl⁻, Br⁻, BF₄⁻, PF₆⁻, SbF₆⁻, CH₃SO₃⁻, 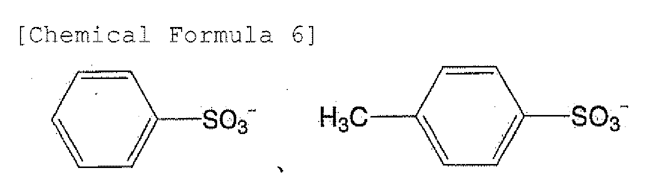 to be readily used.

Any metal complex having negative charge can also be suitably used as X⁻. Examples of the individual metal complexes include transition metal chelates such as acetylacetonate chelates, azos, salicylaldehyde oximes, diimmoniums, dithiols, dipyromethenes, squaliliums, thiocatechol chelates, thiobisphenolate chelates, bisdithio-α-diketone chelates, bisphenylenedithiols, and formazans. Among these complexes preferred are azo metal complexes from the viewpoint of light resistance and solubility in solvents. Examples of the transition elements in the metal complexes illustrated above include vanadium, niobium, tantalum, chromium, molybdenum, tungsten, manganese, technetium, rhenium, iron, ruthenium, osmium, cobalt, rhodium, iridium, nickel, palladium, platinum, copper, silver, gold, zinc, cadmium, and mercury. Among these elements preferred are vanadium, manganese, cobalt, and copper from the viewpoint of economic efficiency and influence on living organisms.

Preferred anions in the azo metal complex as X⁻ include anions in azo-metal complexes represented by the following general formula [II].

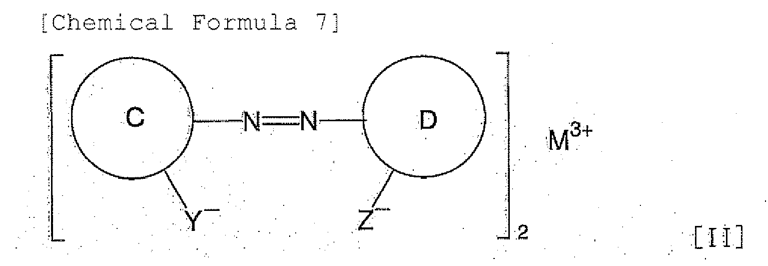

Rings C and D in general formula [II] each independently represent an aromatic ring or a heterocycle, provided that at least one of the rings C and D represents a heterocycle. Y and Z each independently represent a group having an active hydrogen. M represents a trivalent metal element. The active hydrogens are dissociated from Y and Z respectively to provide an azo ligand having a charge of -2. Two azo ligands and one metal atom having a charge of +3 can form a metal complex having a charge of -1 as a whole.

The rings represented by C and D in general formula [II] may be aromatic rings or unsaturated or saturated heterocycles, and have further substituents other than Y and Z. The rings represented by C and D may be of any type, but examples include aromatic rings such as benzene and naphthalene; unsaturated heterocycles such as pyridine, pyrimidine, and thiophene; saturated heterocycles such as Meldrum's acid, barbituric acid, and pyridone.

The groups represented by Y and Z in general formula [II] may be any group having an active hydrogen, proton being dissociated to charge the group negatively. The groups represented by Y and Z may be of any type, but examples include carboxylic acids, sulfonic acids, amino groups, hydroxyl groups, amido groups, boronic acids, and phosphorus acids. The group may be included within a ring.

The element represented by M in general formula [II] represents a trivalent metal element. A metal complex having one metal element coordinated with two tridentate ligands is believed to be stabilized in the hexadentate form, assuming that the groups Y and Z and one of nitrogen atoms in the azo bond in the azo ligand are coordinated to the metal. Therefore, an azo-metal complex formed of two ligands each having a charge of -2 and one metal having a charge of +3 is most stable, and preferably can be an equimolar pair with an azacyanine dye represented by general formula

Among the azo-metal complex anions represented by above-described general formula [II] preferred are, for example, azo metal complex anions represented by following general formulae [II-1] to [II-4].

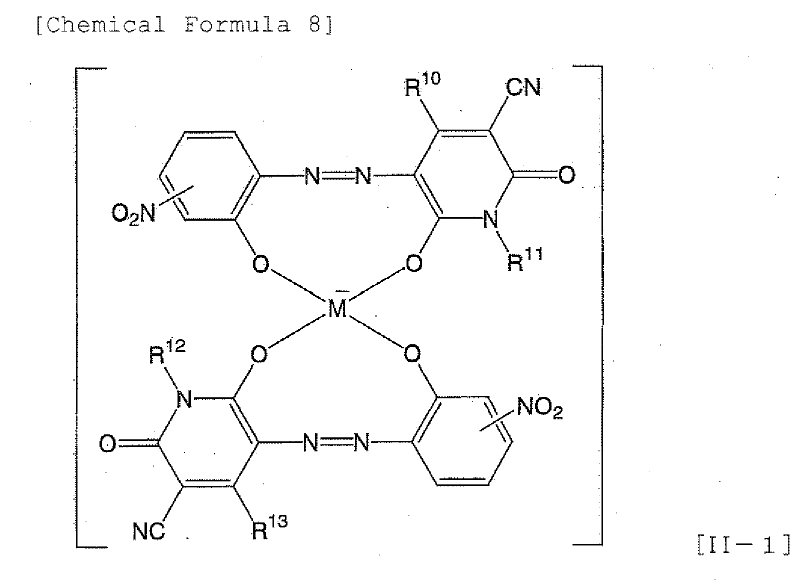

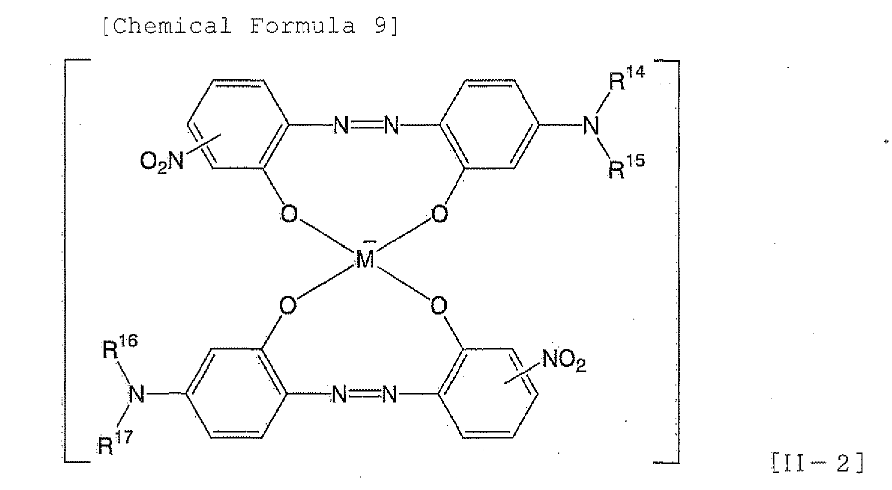

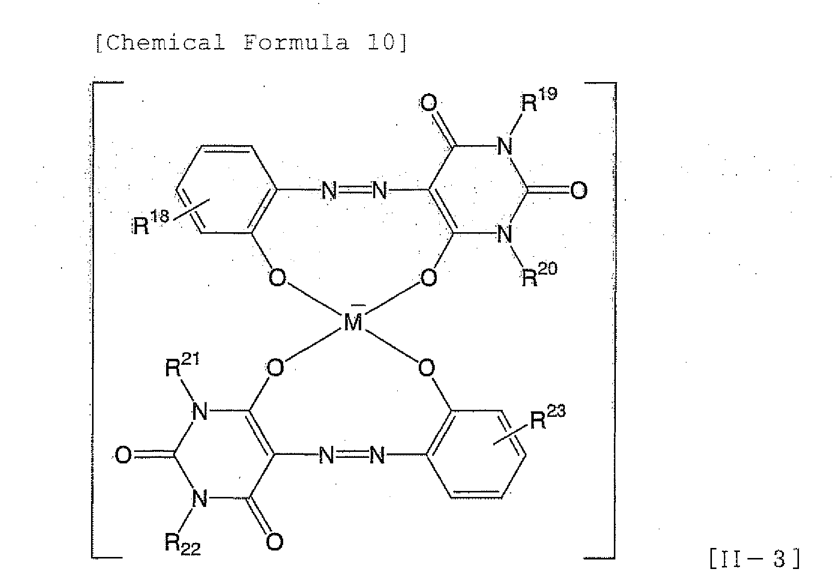

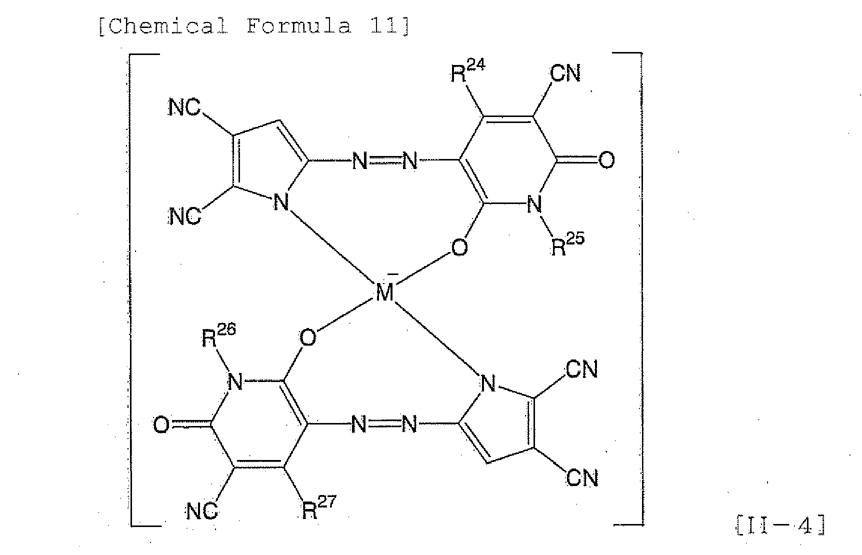

M in above-described general formulae [II-1] to [II-4] represents a transition element belonging to Groups 5 to 12 in the Periodic Table of the Element.

R¹¹, R¹², R¹⁴ to R¹⁷, R¹⁹ to R²², R²⁵, and R²⁶ in above-described general formulae [II-1] to [II-4] represent hydrocarbon groups. These hydrocarbon groups in one molecule may be the same or different from each other. Individual hydrocarbon groups may be of any type, for example, aliphatic hydrocarbon groups or alicyclic hydrocarbon groups. Examples of the aliphatic hydrocarbon groups include straight or branched aliphatic hydrocarbon groups having one to six carbon atoms, for example, methyl group, ethyl group, propyl group, isopropyl group, isopropenyl group, 1-propenyl group, 2-propenyl group, 2-propynyl group, butyl group, isobutyl group, sec-butyl group, tert-butyl group, 2-butenyl group, 1,3-butadienyl group, pentyl group, isopentyl group, neopentyl group, tert-pentyl group, 1-methylpentyl group, 2-methylpentyl group, and 2-penten-4-inyl group. Examples of the alicyclic hydrocarbon groups include cyclopropyl group, cyclobutyl group, cyclopentyl group, cyclohexyl group, and cyclohexenyl group. One or more hydrogen atoms in such hydrocarbon groups may be substituted by halogen atoms such as fluorine atom, chlorine atom, and bromine atom. The azacyanine dyes of formula [I] containing azo-metal complex anions of above-described general formulae [II-1] to [II-4] typically have increased solubility in solvents as the carbon numbers of R¹¹, R¹², R¹⁴ to R¹⁷, R¹⁹ to R²², R²⁵, and R²⁶ increase, although the solubility depends on the solvent.

R¹⁰, R¹³, R¹⁸, R²³, R²⁴ and R²⁷ in above-described general formulae [II-1], [II-3], and [II-4] represent hydrogen atoms or substituents. These substituents in one molecule may be the same or different from each other. Examples of the individual substituent include aliphatic hydrocarbon groups such as methyl group, ethyl group, propyl group, isopropyl group, isopropenyl group, 1-propenyl group, 2-propenyl group, 2-propynyl group, butyl group, isobutyl group, sec-butyl group, tert--butyl group, 2-butenyl group, 1,3-butadienyl group, pentyl group, isopentyl group, neopentyl group, tert-pentyl group, 1-methylpentyl group, 2-methylpentyl group, and 2-penten-4-inyl group; alicyclic hydrocarbon groups such as cyclopropyl group, cyclobutyl group, cyclopentyl group, cyclohexyl group, and cyclohexenyl group; aromatic hydrocarbon groups such as phenyl group, o-tolyl group, m-tolyl group, p-tolyl group, xylyl group, mesityl group, o-cumenyl group, m-cumenyl group, p-cumenyl group, and biphenylyl group; ether groups such as methoxy group, ethoxy group, propoxy group, isopropoxy group, butoxy group, isobutoxy group, sec-butoxy group, tert-butoxy group, pentyloxy group, and phenoxy group; ester groups such as methoxycarbonyl group, ethoxycarbonyl group, propoxycarbonyl group, acetoxy group, and benzoyloxy group; amino groups such as dimethylamino group, diethylamino group, dipropylamino group, diisopropylamino group, dibutylamino group, and dipentylamino group; halogen atoms such as fluorine atom, chlorine atom, bromine atom, and iodine atom; hydroxy group, carboxy group, cyano group, nitro group; and substituents formed by combining several substituents described above.

In the azo metal complex anion represented by above-described general formulae [II-1] and [II-2], two nitro groups may be located at an ortho, meta, or para position relative to the azo group, but preferably located at a meta position from the viewpoint of synthesis.

The above-described azacyanine dye of formula [I] has many positions to which substituents can be introduced, and has characteristics of easy adjustment of the wavelength because electron-attracting or electron-releasing effects of the substituents are readily conducted through the conjugated π-electron system. For example, when R⁵ represents an aromatic ring group or a heterocycle group or when R⁴ and R⁵ taken together form a ring, appropriate selection of substituents can change the wavelength of absorption maximum from about 350 nm up to 450 nm. This can provide higher absorption in recording light wavelength, resulting in improvement in recording sensitivity.

Preferred examples of the azacyanine dye of formula [I] include compounds illustrated by following structural formulae (1) to (3), (5), (6), (8) to (16), and (20) to (37) (hereinafter, the compounds illustrated by the structural formulae are each abbreviated as "Exemplary Compound (1)" etc. assigned the number of the respective structural formula). However, the following compounds are merely illustrative, and are not intended to limit the azacyanine dye of formula [I] that can be used in an optical recording medium of the present invention.

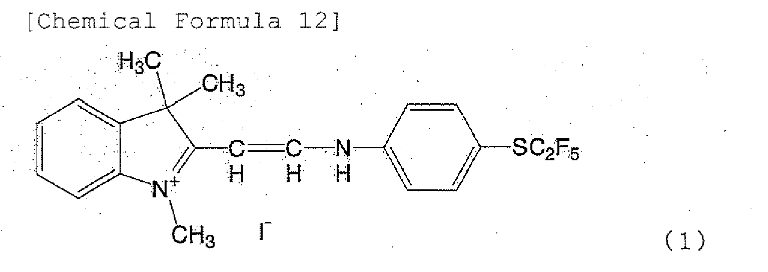

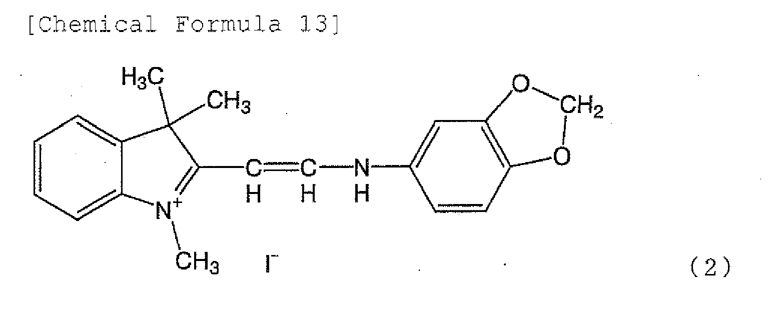

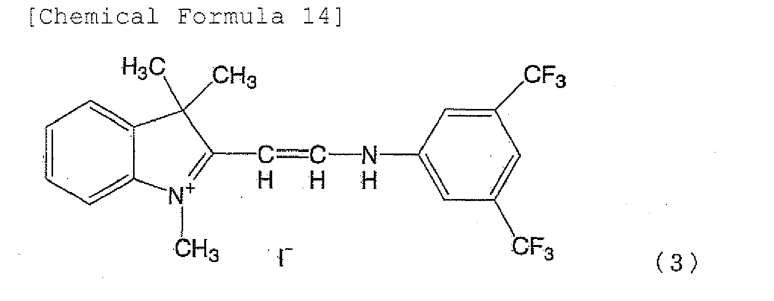

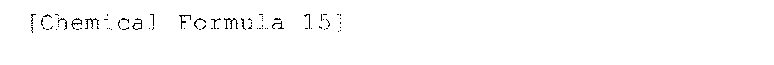

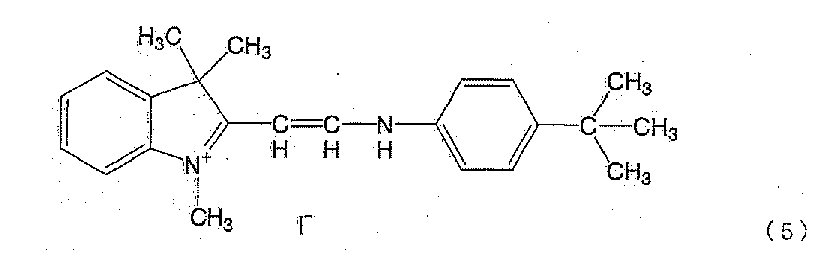

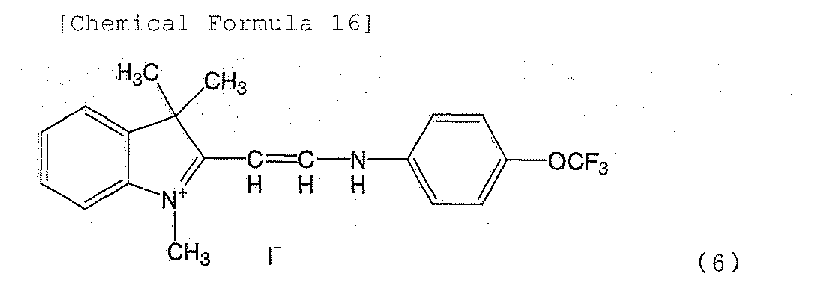

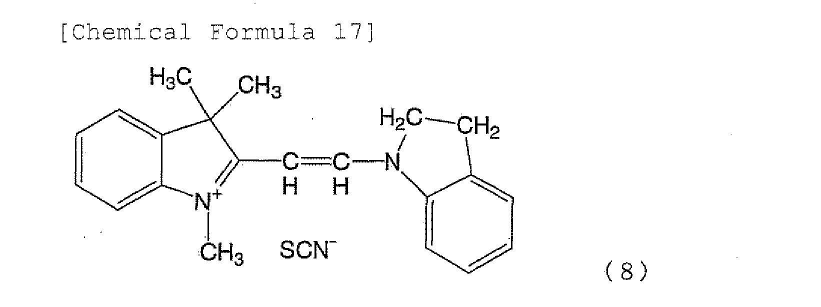

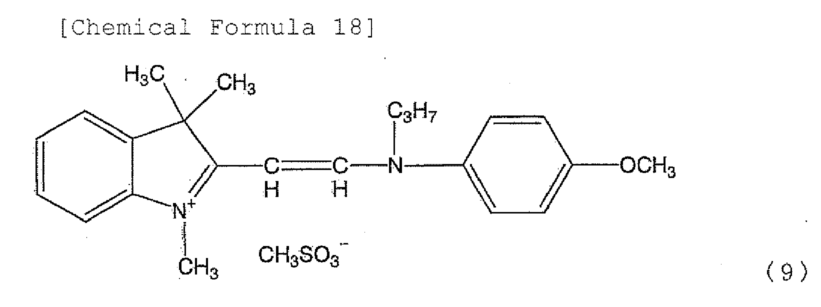

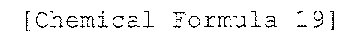

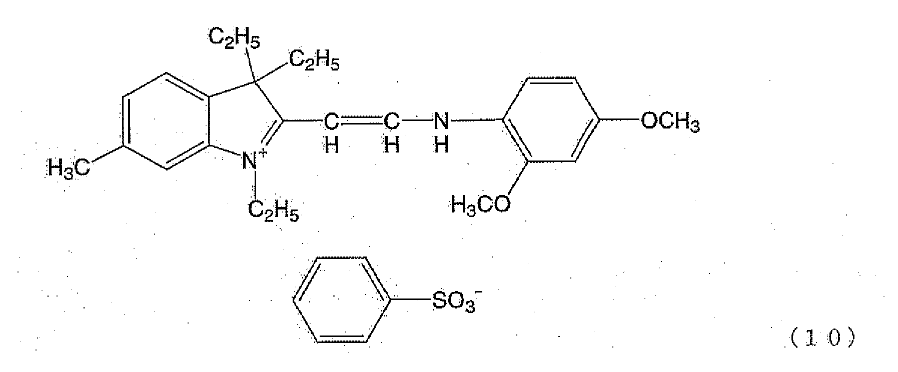

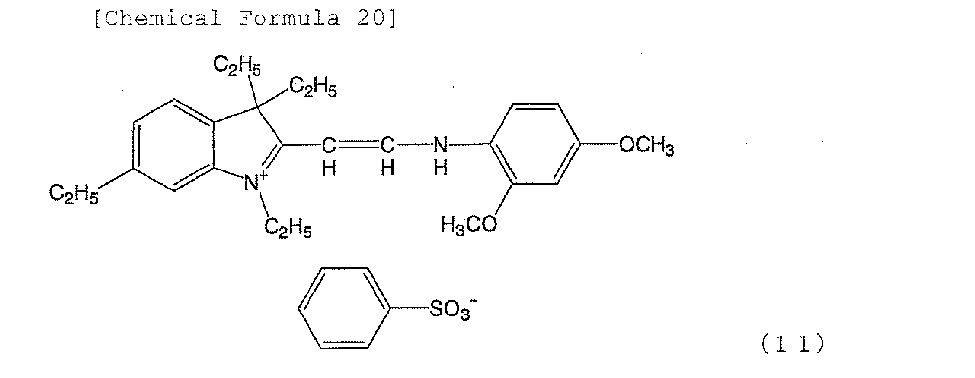

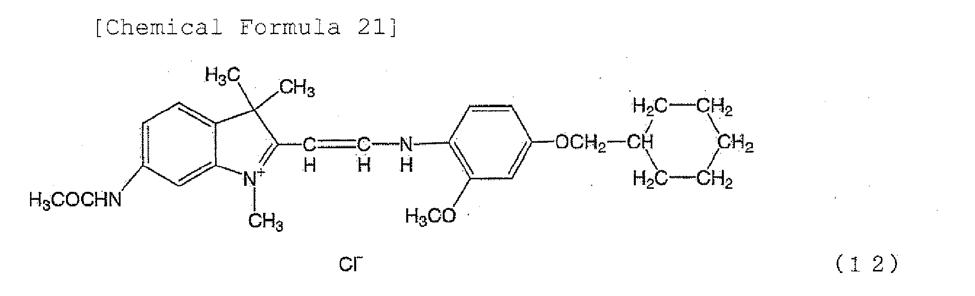

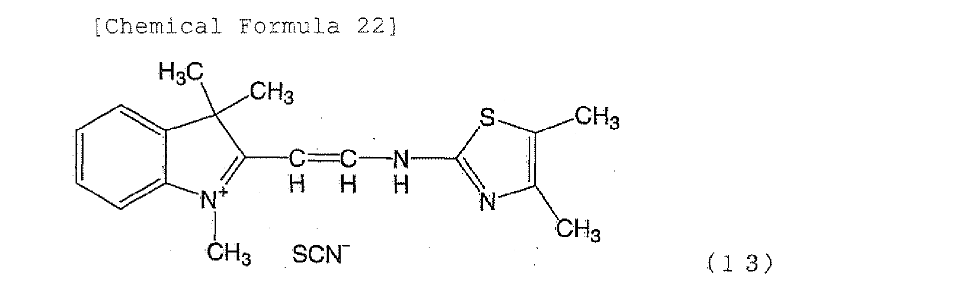

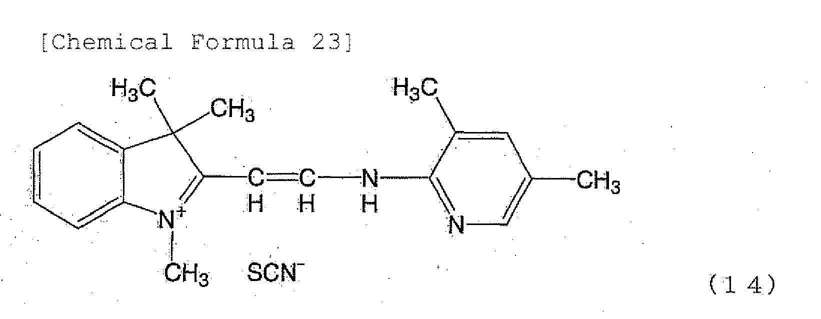

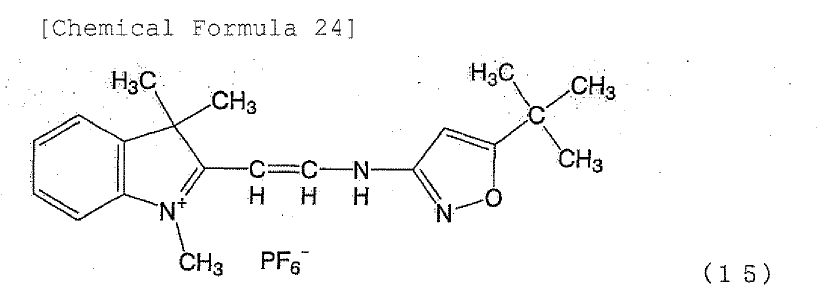

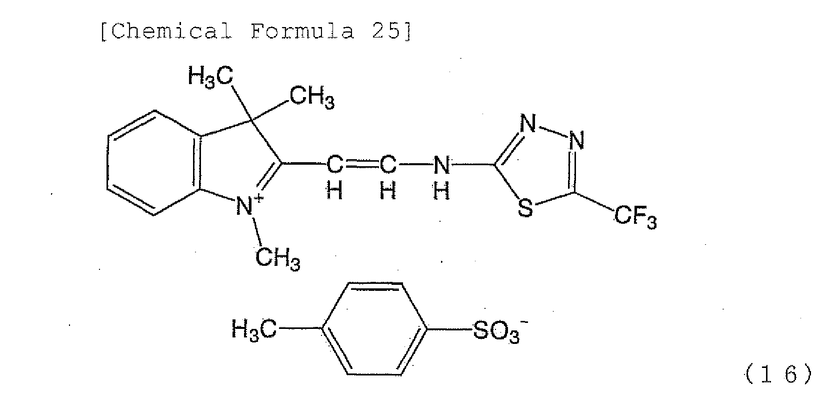

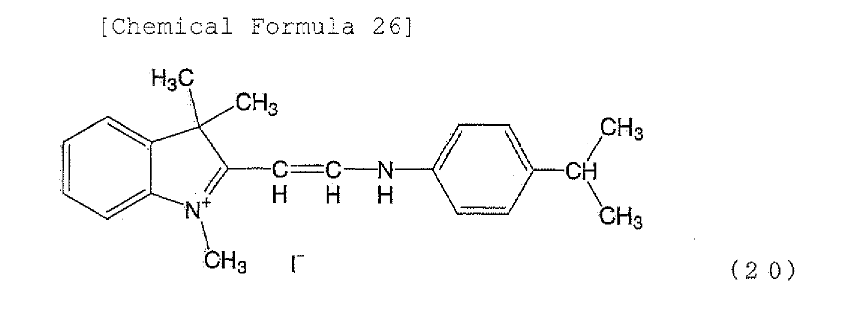

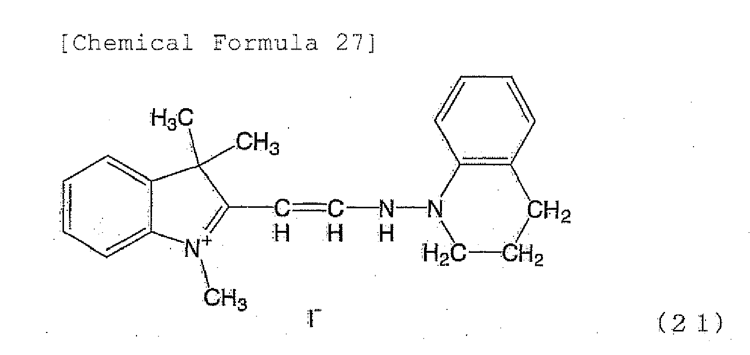

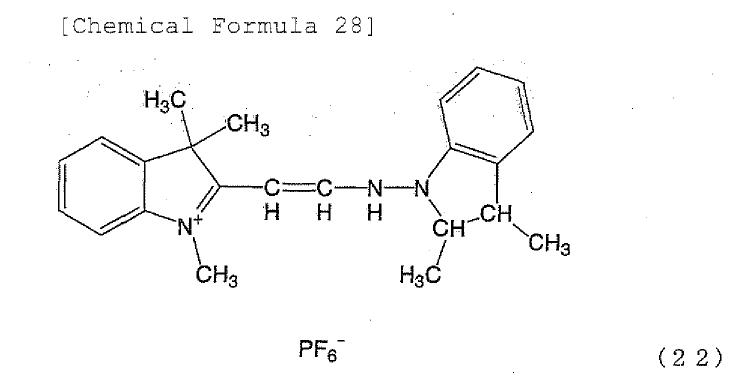

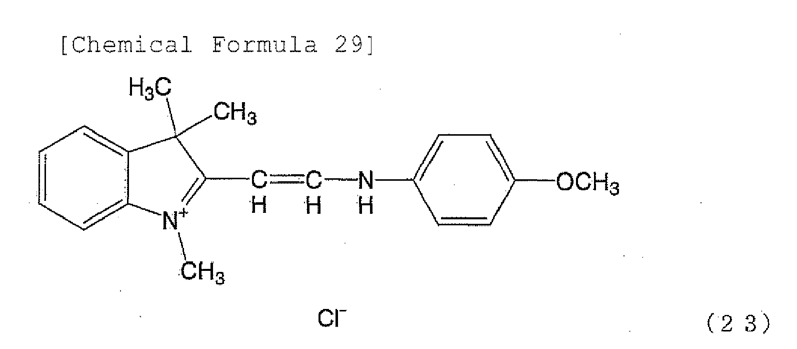

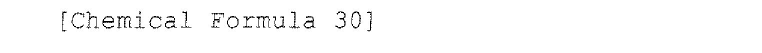

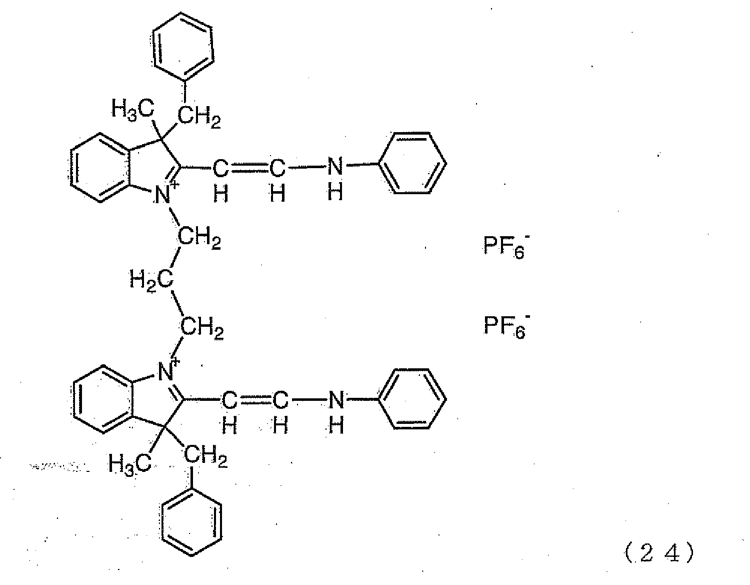

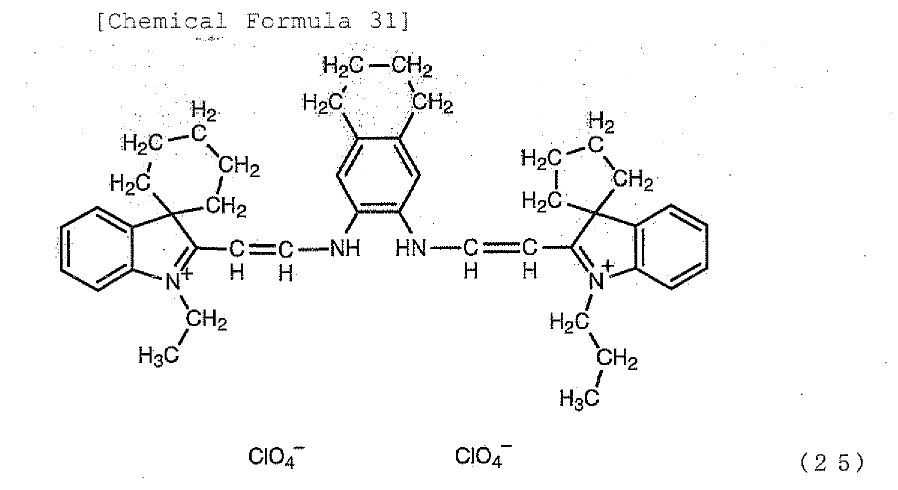

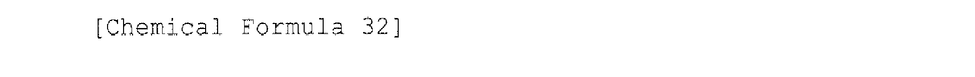

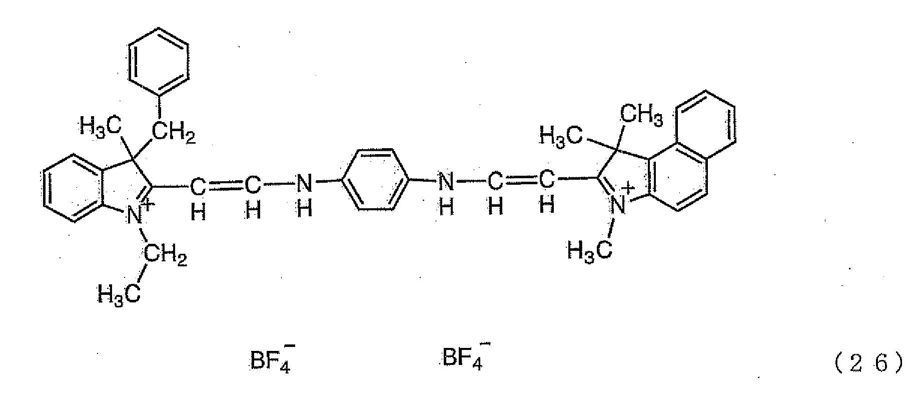

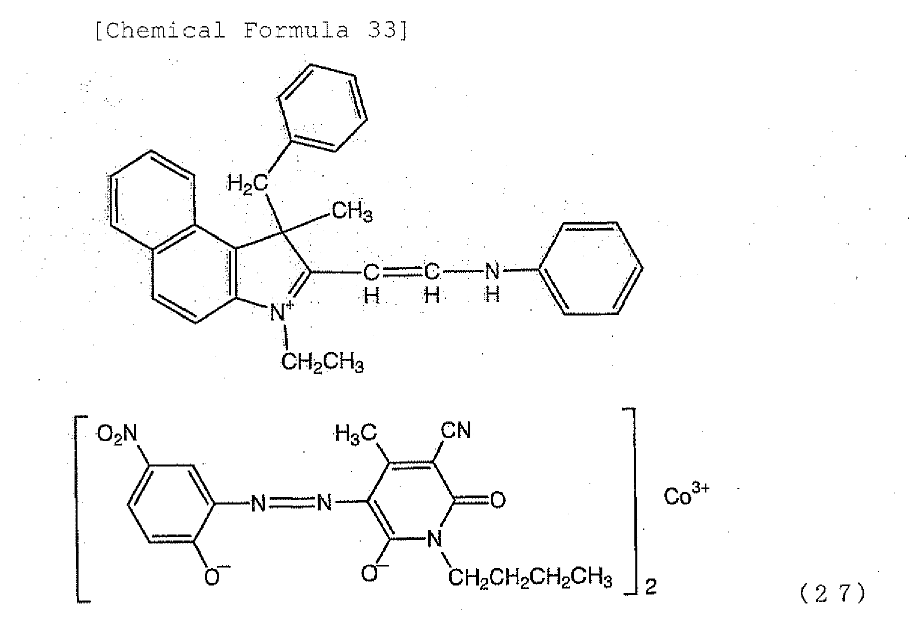

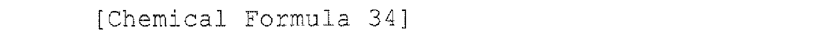

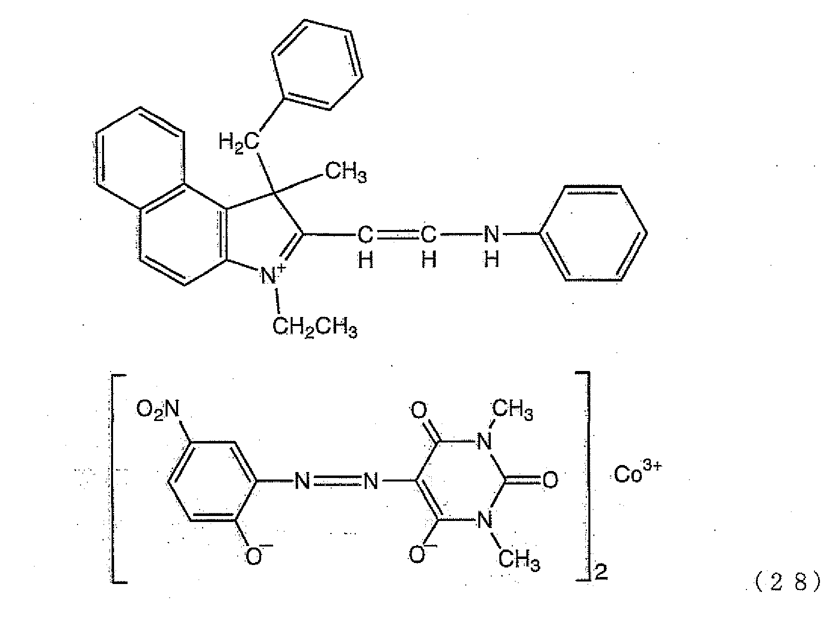

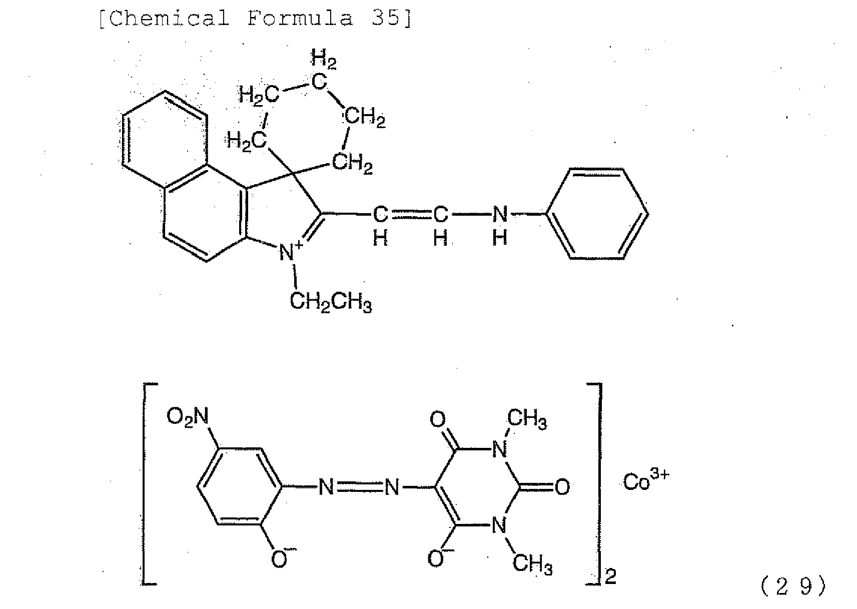

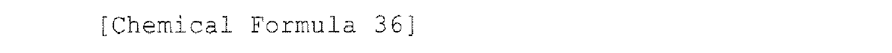

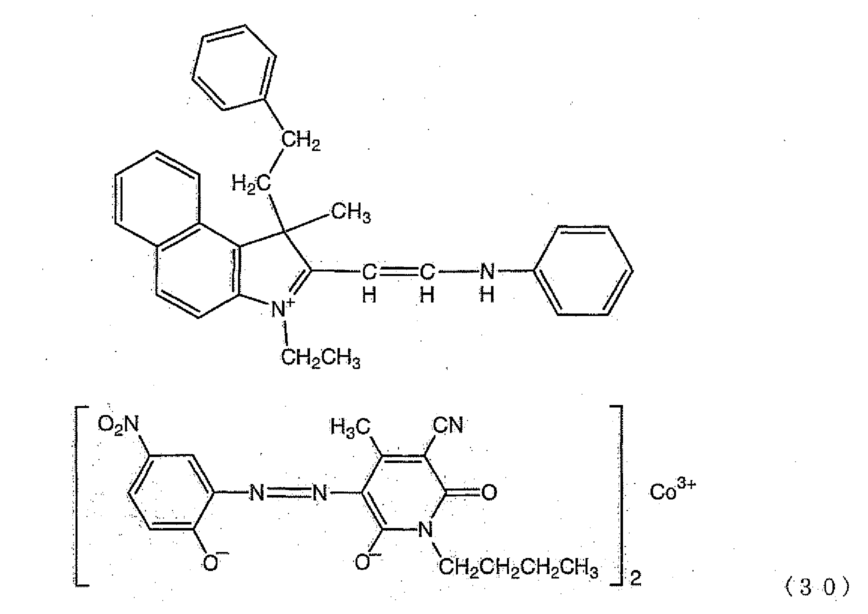

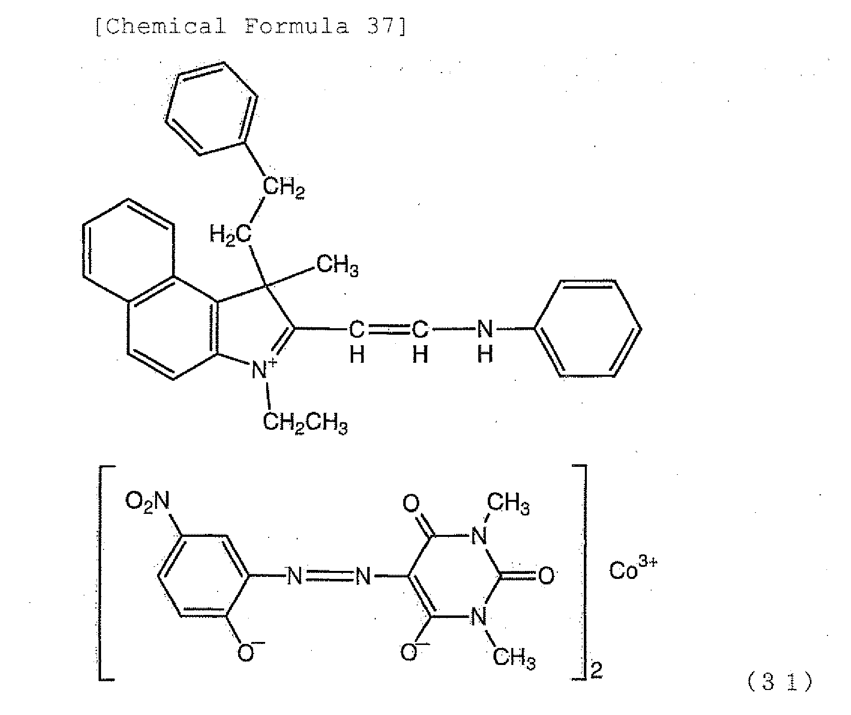

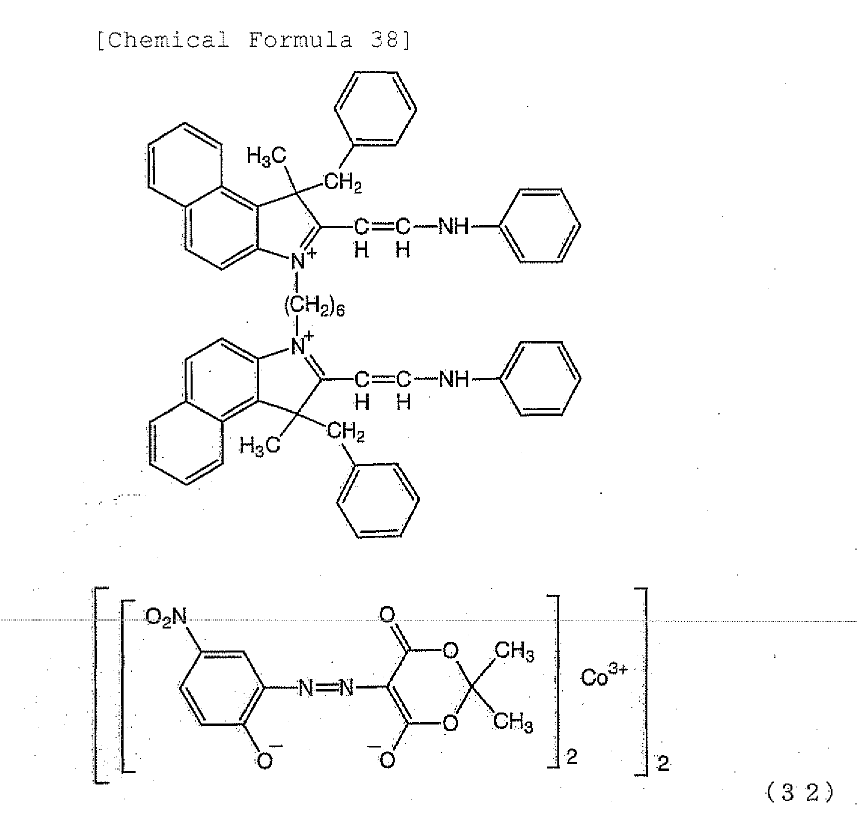

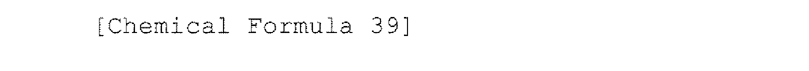

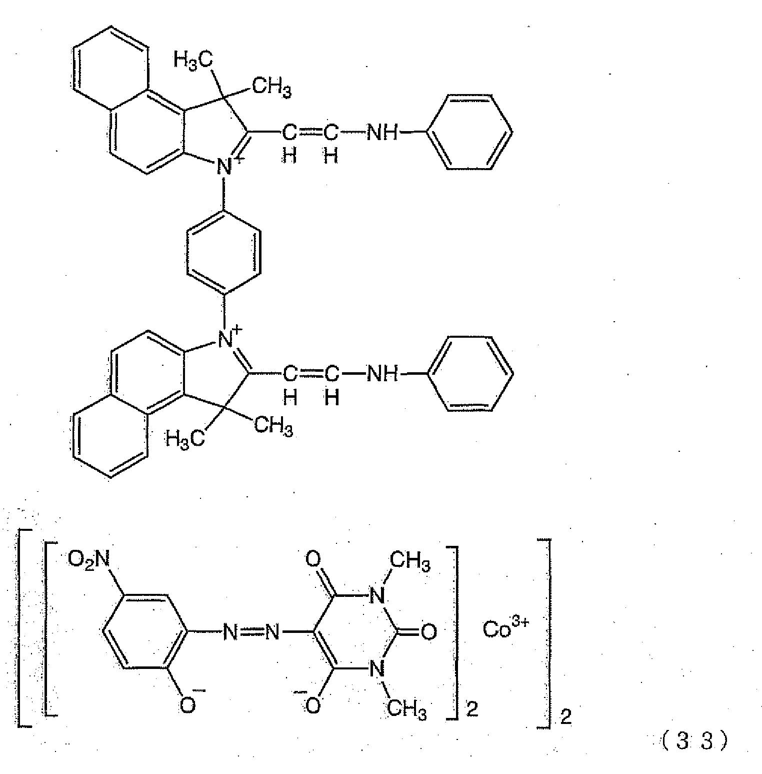

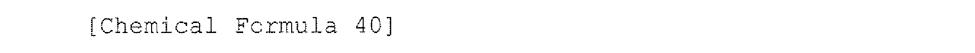

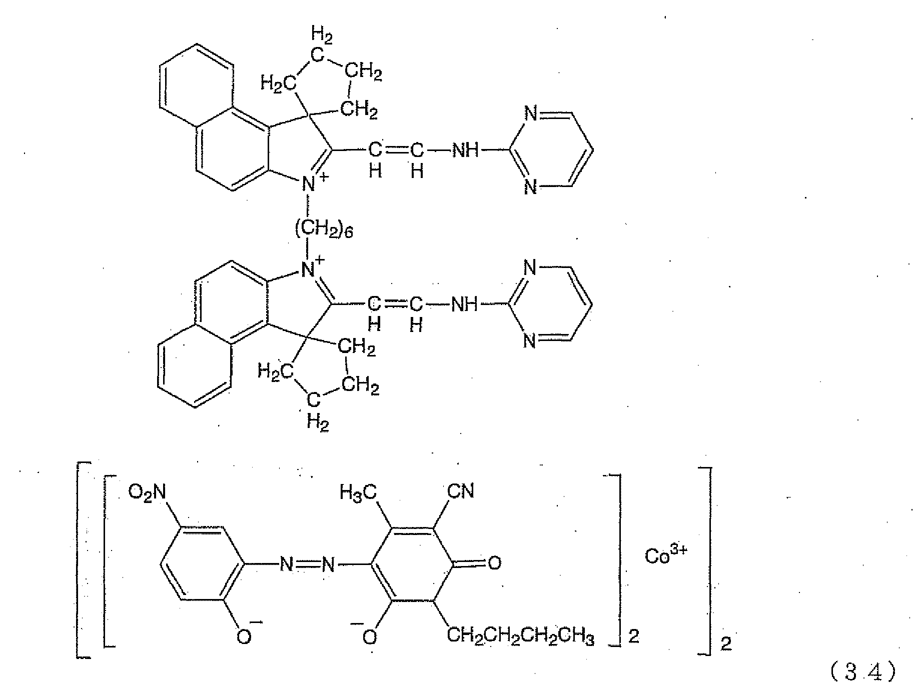

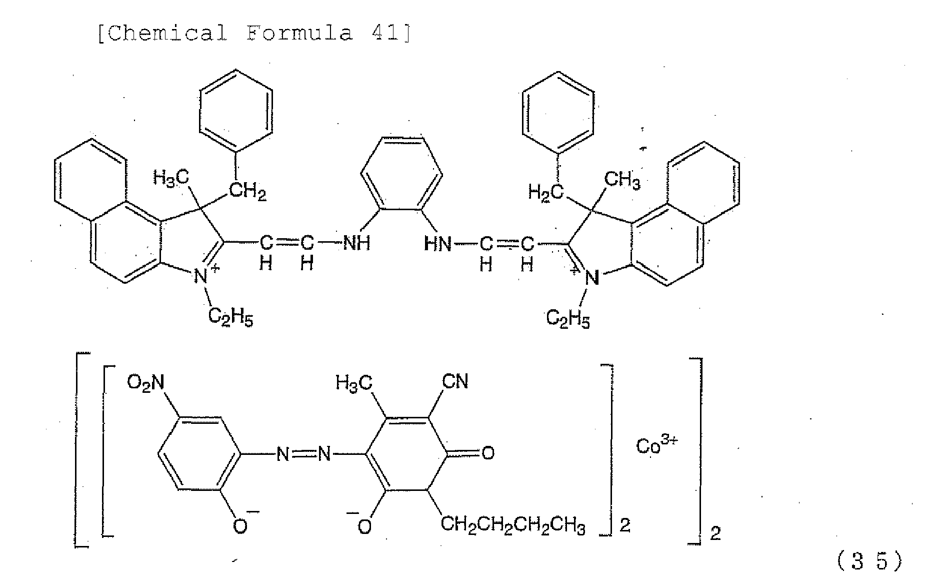

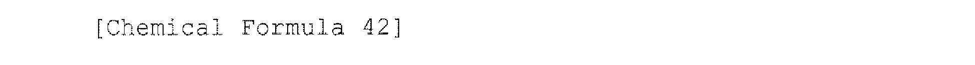

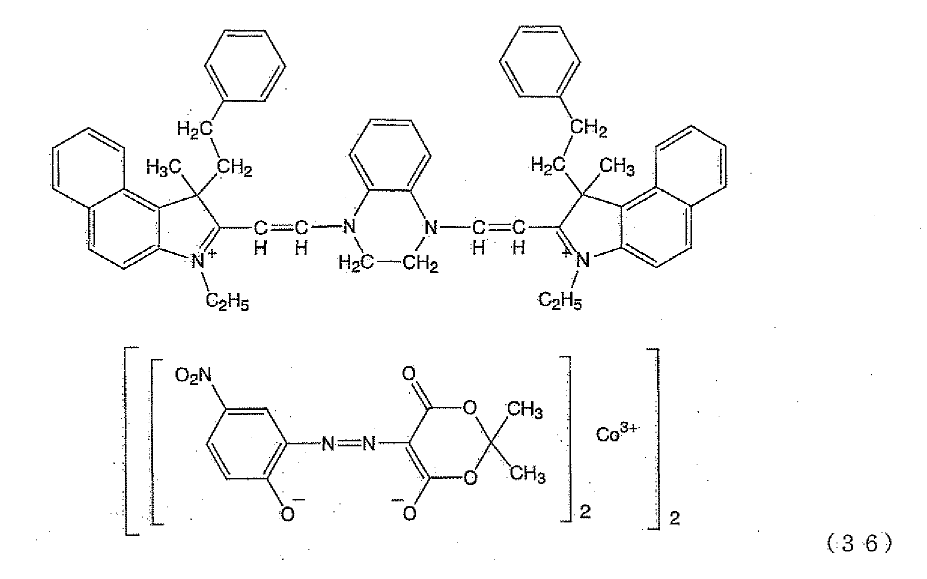

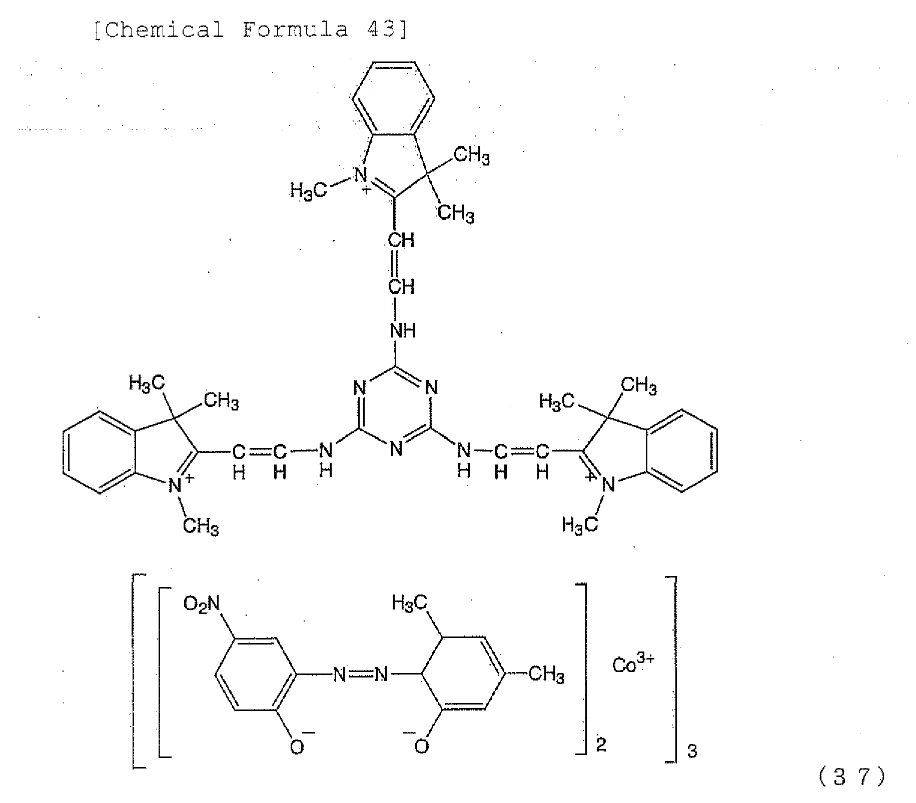

The compound having a thiazole ring group as R⁵, which is shown as Exemplary Compound (13), is preferred because it is expected to have improved solubility.
The compound having a heterocycle and a benzene ring fused to the heterocycle as R⁵, which is shown as Exemplary Compound (8), has an advantage of increased stability as a dye due to its decreased hydrophilicity (its reduced hydrolyzability).

Among Exemplary Compounds (1) to (3), (5), (6), (8) to (16), and (20) to (37), preferred are Exemplary Compounds (1) to (3), (6), (15), and (20) to (23) because they can more significantly exhibit the above-described effects when used in the recording layer of an optical recording medium. Exemplary Compounds (24) to (30) are also preferred.

In particular, an azacyanine dye represented by following formula (i) (called "azacyanine dye of the present invention"), typical examples of which are represented by Exemplary Compounds (27) and (28), is especially preferred because it is a dye compound of a new structure, and can significantly exhibit the above-described effects when used in the recording layer of an optical recording medium.

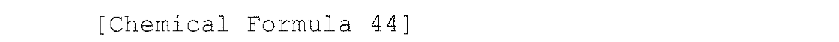

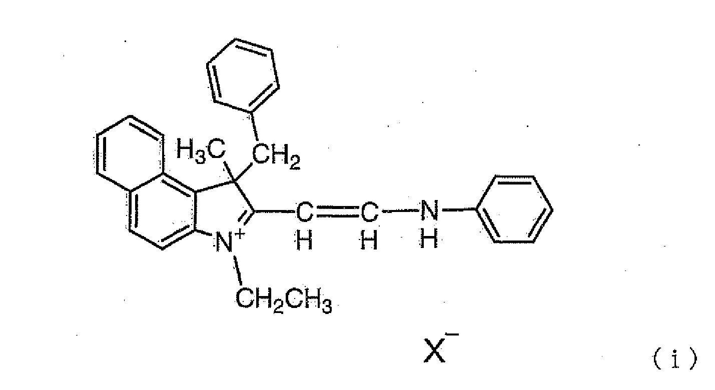

wherein X⁻ represents a counter anion.

The above-described azacyanine dye of formula [I] can be produced by any process, for example, a process including heating 2-(l,3,3-trimethylindolin-2-ylidene)acetaldehyde and an amine compound in acetic acid, followed by discharge of the reaction mixture into water for salt precipitation (See, for example, K. Venkataraman, The Chemistry of Synthetic Dyes Volume II, 1952, Academic Press, p.1175).

### [II. Optical Recording Media]

The optical recording medium of the present invention includes at least a substrate and a recording layer thereon that can record or read information by irradiation with light, the recording layer containing an azacyanine dye of above-described general formula [1]. In addition to the substrate and the recording layer, the medium may further have additional layers such as a primer layer, a reflective layer, and a protective layer, if necessary.

The optical recording medium of the present invention will now be described in further detail by reference to embodiments. However, the following embodiments are merely illustrative, but not limiting the present invention. Any variations of the embodiments can be made within the spirit of the invention.

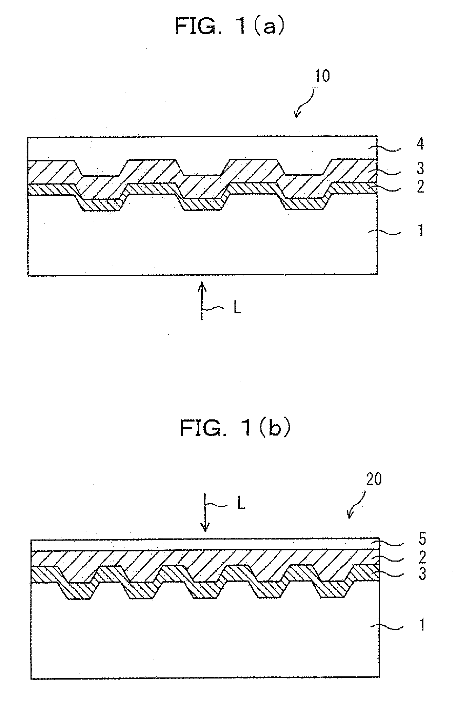

A first embodiment of the present invention will first be described. Fig. 1(a) is a partial cross-sectional view that schematically illustrates an example of a layered structure of an optical recording medium according to the first embodiment of the present invention. An optical recording medium 10 represented by Fig. 1(a) has a structure in which a substrate 1 made of light-transmissive material, a recording layer 2 on the substrate 1, a reflective layer 3 on the recording layer 2, and a protective layer 4 are sequentially stacked. Recording and reading information on the optical recording medium 10 is conducted by irradiating the side of the substrate 1 with a laser beam L.

For convenience, for the optical recording medium 10, the side on which the protective layer 4 is present is referred to as the upper side, the side on which the substrate 1 is present is referred to as the lower side, and each face of any other layer corresponding to these sides is referred to as the upper face and the lower face of this layer, respectively.

The substrate 1 can contain various materials that are essentially transparent in the wavelength range of recording light and reading light, particularly including resins, for example, acrylic resins, methacrylic resins, polycarbonate resins, polyolefin resins (especially, amorphous polyolefins), polyester resins, polystyrene resins, and epoxy resins; and glass. The substrate may have a structure of a resin layer made of a radiation-curable resin such as a light-curable resin provided on glass. Among the materials preferred are polycarbonate resins used in injection molding from the viewpoint of high productivity, cost, and hygroscopicity resistance, and amorphous polyolefins from the viewpoint of chemical resistance and hygroscopicity resistance. Glass is also preferred from the viewpoint of quick response.

When a resin substrate 1 or a substrate 1 having a resin layer provided on the side of the recording layer (at the upper side) is used, guide grooves or pits for recording and reading light may be formed on the upper face of the substrate. The guide grooves are arrayed into a concentric or a spiral shape from the center of the optical recording medium 10. When spiral guide grooves are formed, the groove pitch is preferably about 0.2 to 1.2 µm.

The recording layer 2 is formed directly on the upper side of the substrate 1, or on the upper side of the primer layer, if needed, provided on the substrate [I], and contains an azacyanine dye of above-described formula [I]. The azacyanine dye of formula [I] may be used either alone or in any combination thereof at any proportion.

The recording layer 2 further contains other classes of dye in addition to an azacyanine dye of formula [I]. Any other classes of dye that mainly have appropriate absorption in the wavelength region of oscillation for a recording laser beam can be used without limitation. The recording layer 2 can also contain a dye used for CD-Rs, which is suitable for recording and reading using a near-infrared laser beam having oscillation wavelength in a wavelength band of 770 to 830 nm, or a dye used for DVD-R, which is suitable for recording and reading using a red laser beam having oscillation wavelength in a wavelength band of 620 to 690 nm together with an azacyanine dye of formula [I]. This can also produce an optical recording medium 10 for recording and reading using several types of laser beam in different wavelength bands. Out of the above-described dyes for CD-Rs or DVD-Rs, one having high light resistance can be selected, and used together with an azacyanine dye of formula [I], resulting in a further improvement in light resistance of the recording medium.

Examples of the other classes of dye other than the azacyanine dye of formula [I] include metal-containing azo dyes, benzophenone dyes, phthalocyanine dyes, naphthalocyanine dyes, cyanine dye, azo dyes, squalilium dyes, metal-containing indoaniline dyes, triarylmethane dyes, merocyanine dyes, azulenium dyes, naphthoquinone dyes, anthraquinone dyes, indophenol dyes, xanthene dyes, oxazine dyes, and pyrylium dyes.

In addition to an azacyanine dye of formula [I], the recording layer 2 can also contain a transition metal chelate compound (for example, acetylacetonate chelate, bisphenyldithiol, salicylaldehyde oxime, or bisdithio-α-diketone) as a single oxygen quencher in order to improve stability and light resistance, or a recording-sensitivity improver such as a metallic compound in order to improve recording sensitivity. The term "metallic compound" used herein refers to a compound containing a metal such as a transition metal in the atomic, ionic, or cluster form, and include compounds, for example, ethylenediamine complexes, azomethine complexes, phenylhydroxyamine complexes, phenanthroline complexes, dihydroxyazobenzene complexes, dioxime complexes, nitrosoaminophenol complexes, pyridyltriazine complexes, acetylacetonate complexes, metallocene complexes, and porphyrin complexes. Any metal atom can be contained, but transition metals are preferred.

A binder, a leveling agent, and an antifoaming agent can also be used if necessary. Preferred binders include polyvinyl alcohol, polyvinyl pyrrolidone, nitrocellulose, cellulose acetates, ketone resins, acrylic resins, polystyrene resins, urethane resins, polyvinyl butyral, polycarbonates, and polyolefins. These may be used either alone or in any combination thereof at any proportion.

The method of forming the recording layer 2 include various common methods of forming thin films such as vacuum deposition, sputtering, doctor blade coating, casting, spin coating, and dipping. Spin coating is preferred from the viewpoint of mass production and cost, and vacuum deposition is preferred to coating because a recording layer 2 having a uniform thickness can be prepared. When a film is formed by spin coating, the number of the spinning operations is preferably 500 to 15000 rpm. Optionally, treatments such as heating or exposure to solvent vapor may be employed after spin coating.

When a recording layer 2 is formed by coating methods such as doctor blade coating, casting, spin coating, and dipping, any coating solvent that does not erode the substrate 1 can be used for dissolving an azacyanine dye of formula [I] to coat the substrate 1. In particular, the coating solvent includes, for example, ketone alcohol solvents such as diacetone alcohol, and 3-hydroxy-3-methyl-2-butanone; cellosolve solvents such as methyl cellosolve, and ethyl cellosolve; chain hydrocarbon solvents such as n-hexane and n-octane; cyclic hydrocarbon solvents such as cyclohexane, methylcyclohexane, ethylcyclohexane, dimethylcyclohexane, n-butylcyclohexane, tert-butylcyclohexane, and cyclooctane; perfluoroalkyl alcohol solvents such as tetrafluoropropanol, octafluoropentanol, and hexafluorobutanol; hydroxycarboxylic acid ester solvents such as methyl lactate, ethyl lactate, and methyl 2-hydroxyisobutyrate. These solvents may be used either alone or in any combination thereof at any proportion.

The recording layer 2 is formed by vacuum deposition, for example, by placing recording layer components including an azacyanine dye of formula [I] and, if necessary, other dyes and various additives into a crucible arranged within a vacuum vessel, evacuating the vacuum vessel to about 10⁻² to 10⁻⁵ Pa by an appropriate vacuum pump, and heating the crucible to evaporate the recording layer components such that the components are deposited on a substrate facing the crucible.

The recording layer 2 may have any thickness because its appropriate film thickness depends on the recording method. However, the recording layer 2 requires a certain thickness to ensure recording, typically at least 1 nm, and preferably 5 nm or more. In this case, the thickness is also typically 300 nm or less, preferably 200 nm or less, and more preferably 100 nm or less since a large thickness may preclude satisfactory recording.

A reflective layer 3 is formed on the recording layer 2. The thickness of the reflective layer 3 is preferably 50 nm to 300 nm. For the reflective layer 3, materials having a sufficiently high reflectivity in the wavelength range of reading light, for example, metals such as Au, A1, Ag, Cu, Ti, Cr, Ni, Pt, Ta, and Pd, may be used either alone or in alloy form. Among these, Au, A1, and Ag are suitable due to high reflectivity as the material for the reflective layer 3. Also, these metals may be contained as the primary component with other materials. The term "primary component" used herein means a material the content of which is 50 weight% or more. The other materials other than the primary component include metals such as Mg, Se, Hf, V, Nb, Ru, W, Mn, Re, Fe, Co, Rh, Tr, Cu, Zn, Cd, Ga, In, Si, Ge, Te, Pb, Po, Sn, Bi, Ta, Ti, Pt, Pd, and Nd, and semimetals. Use of Ag is especially preferred as the primary component, because of low cost, high reflectivity, and beautiful white ground-color when a print-receptive layer described below is provided. For example, an alloy containing Ag and about 0.1 atom% to 5 atom% of one or more selected from Au, Pd, Pt, Cu, and Nd, is preferred due to high reflectivity, high endurance, high sensitivity, and low cost. Such metals include, for example, AgPdCu alloy, AgCuAu alloy, AgCuAuNd alloy, and AgCuNd alloy. Materials other than metals can also be used by alternately stacking low-refractive index thin layers and high-refractive index thin layers to form a multi-layered film as the reflective layer 3.

Examples of the process for preparation of a reflective layer 3 include sputtering, ion plating, chemical vapor deposition, and vacuum deposition. A well-known inorganic or organic intermediate layer or adhesive layer can also be provided over the substrate 1 or under the reflective layer 3 in order to improve reflectivity, recording characteristics, and adhesiveness.

A protective layer 4 is formed on the reflective layer 3. For the protective layer 4, any material that protects the reflective layer 3 from any external force can be used. The organic material can include thermoplastic resins, thermosetting resins, electron-radiation curing resins, ultraviolet (hereinafter referred to as "UV") curing resins. The inorganic materials include silicon oxide, silicon nitride, magnesium fluoride (MgF₂), and tin oxide (Sn0₂)

The protective layer 4 can be formed by dissolving a thermoplastic resin or a thermosetting resin in an appropriate solvent to prepare a coating solution, applying the coating solution onto the reflective layer 3 and drying the applied solution. The protective layer 4 can also be formed by applying an UV curing resin directly on the reflective layer 3, or dissolving the resin in an appropriate solvent to prepare a coating solution and applying the coating solution onto the reflective layer 3, and irradiating the resin with UV light for curing. Examples of the UV curing resin include acrylate resins such as urethane acrylate, epoxy acrylate, and polyester acrylate. These materials may be used either alone or in any mixture thereof. The protective layer may be in the form of a monolayer or a multilayer.

Like the recording layer 2, the protective layer 4 may be formed by any coating processes such as spin coating and casting, or sputtering or chemical vapor deposition. Among them preferred is spin coating. The protective layer 4 has a thickness of typically 0.1 µm or more and preferably 3 µm or more because the layer requires a certain thickness to accomplish its protective function. In this case, the thickness is also typically 100 µm or less and preferably 30 µm since a larger thickness may provide no further effect, and take a longer time to form the protective layer 4 with higher cost.

The optical recording medium 10 has been described with reference to a structure formed by sequentially stacking the substrate, the recording layer, the reflective layer, and the protective layer by way of example, but may have any other layer structure.

For example, another substrate 1 may further be laminated on the upper face of the protective layer 4 in the above exemplary layer structure, or on the upper face of the reflective layer 3 in the above exemplary layer structure without the protective layer 4. In this case, the substrate 1 may be a bare substrate with no other layer, or a substrate having any layer such as a reflective layer 3 on its laminating face or the opposite face. Likewise, two optical recording media 10 each having the above exemplary layer structure, or two optical recording media 10 each having the above exemplary layer structure without the protective layer 4 may be laminated respectively facing the upper face of each protective layer 4 and/or the upper face of each reflective layer 3 each other.

A second embodiment of the present invention will now be described. Fig. 1(b) is a partial cross-sectional view that schematically illustrates an exemplary layered structure of an optical recording medium according to a second embodiment of the present invention. Components in Fig. 1(b) which are identical to those in Fig. 1(a) are assigned the same symbols, without explanation. An optical recording medium 20 represented by Fig. 1(b) has a structure in which a substrate 1 made of light-transmissive material, a reflective layer 3 on the substrate 1, a recording layer 2 on the reflective layer 3, and a protective coating 5 are sequentially stacked. Recording and reading information on the optical recording medium 20 is conducted by irradiating the side of the protective coating 5 with the laser beam L.

The protective coating 5 may be a film or sheet form bonded with an adhesive, or may be formed by applying a film-forming solution containing the material for the protective layer 4 described above followed by curing or drying the solution. The protective coating 5 has a thickness of typically 0.1 µm or more and preferably 3 µm or more because the coating requires a certain thickness to accomplish its protective function. In this case, the thickness is also typically 300 µm or less and preferably 200 µm or less since a larger thickness may provide no improved effect, and take a longer time to form the protective coating 5 with higher cost.

Each of the recording layer 2 and the reflective layer 3 can typically employ the same medium as the optical recording medium 10 of the first embodiment. However, the substrate 1 in the second embodiment need not to be transparent, and therefore opaque resins, ceramics, metals (including alloys) can be used in addition to the above-described materials. Such layered structure may have any layer between each layer described above that does not impair the feature of the present invention.

One means of increasing the recording densities of the optical recording media 10 and 20 may be to increase the numerical aperture of an object lens. This can form a micro light spot on the information recording face to which a laser beam is converged. However, the higher the numerical aperture of the object lens tends to yield the higher the light spot aberration due to warping of the optical recording media 10 and 20 during irradiation with the laser beam for recording and reading. This may preclude stable generation of satisfactory recording and reading signals.

A larger thickness of the transparent substrate or protective coating which transmits a laser beam increases such aberration. For reducing the aberration, it is preferred to thin the substrate or the protective coating as much as possible. However, the substrate 1 typically requires a certain thickness to ensure the strength of the optical recording media 10 and 20. In this case, the structure of the optical recording medium 20 of the second embodiment (an optical recording medium 20 having a basic layered structure composed of a substrate 1, a reflective layer 3, a recording layer 2, and a protective coating 5) is preferably employed. A thinner protective coating 5 of an optical recording medium 20 according to the second embodiment can be prepared more easily than a thinner substrate 1 of an optical recording medium 10 according to the first embodiment, and therefore the optical recording medium 20 of the second embodiment is preferably used.

However, the structure of an optical recording medium 10 of the first embodiment (an optical recording medium 10 having a basic layered structure composed of a substrate 1, a recording layer 2, a reflective layer 3, and a protective layer 4) can also be employed by reducing the thickness of the transparent substrate 1 that transmits laser beams for recording and reading to about 50 to 300 µm in order to reduce the aberration.

An ultraviolet curing resin layer or an inorganic thin film may also be formed on the incident surface of laser beams for recording and reading (typically on the lower face of the substrate 1) after forming each of the other layers for the purpose of protecting the surface or preventing dust from adhering to the surface. Alternatively, a print-receptive layer on which writing or printing can be conducted with various printers such as ink-jet printer and thermal transfer printer, or various writing instruments may be formed on a face other than the incident surface of laser beams for recording and reading (typically on the upper face of the reflective layer 3 or the protective layer 4).

In the optical recording media 10 and 20 according to the embodiments of the present invention, the laser beam used for recording and reading information having a shorter wavelength is preferred from the viewpoint of high-density recording, and especially preferred are laser beams with a wavelength of 350 to 530 nm. Typical examples of such laser beams include laser beams with a center wavelength of 405 nm, 410 nm, and 515 nm.

Laser beams with a wavelength of 350 to 530 nm can be obtained from high-power semiconductor laser beams with blue wavelengths of 405 nm and 410 nm or with a blue-green wavelength of 515 nm. In addition, such beams can be obtained by converting the wavelength of any oscillating laser beams of, for example, (a) continuously oscillating semiconductor laser beams with a fundamental oscillation wavelength of 740 to 960 nm, and (b) continuously oscillating solid laser beams with a fundamental oscillation wavelength of 740 to 960 nm excited by semiconductor laser beams by a second-harmonic generation (SHG) element.

The SHG element may be any piezo element lacking inversion symmetry, and KDP (KH₂P0₄), ADP (NH₄H₂PO₄), BNN (Ba₂NaNbₛ0₁ₛ), KN (KNbO₃), LBO (LiB₃O₅), and compound semiconductors are preferred as materials for the piezo element. Examples of the second harmonics include 430 nm, a harmonic of its fundamental oscillation wavelength in the case of semiconductor laser beams with a fundamental oscillation wavelength of 860 nm, or 430 nm, a harmonic from a Cr-doped LiSrAlF₆ crystal (with a fundamental oscillation wavelength of 860 nm) in the case of solid laser beams excited by semiconductor laser beams.

During recording of information on the optical recording media 10 and 20 according to each embodiment of the present invention, the recording layer 2 is irradiated with laser beams converged into typically about 0.4 to 0.6 µm (typically, transmitted from the side of substrate 1 through the substrate 1). A part of the recording layer 2 is irradiated with laser beams, and absorbs the laser beam energy to bring about thermal changes such as decomposition, exothermicity, and dissolution, which leads to changes in optical properties.

During reding of the information recorded on the recording layer 2, the recording layer 2 is also irradiated with low-energy laser beams (typically from the same direction as that during reading). The information is read by reading the difference in reflectivity between a part where changes in chemical properties have occurred (that is, the part where information has been recorded) and a part with no change in chemical properties, in the recording layer 2.

An optical recording medium having multiple recording layers and a process of producing the same will now be described as a third embodiment of the present invention.

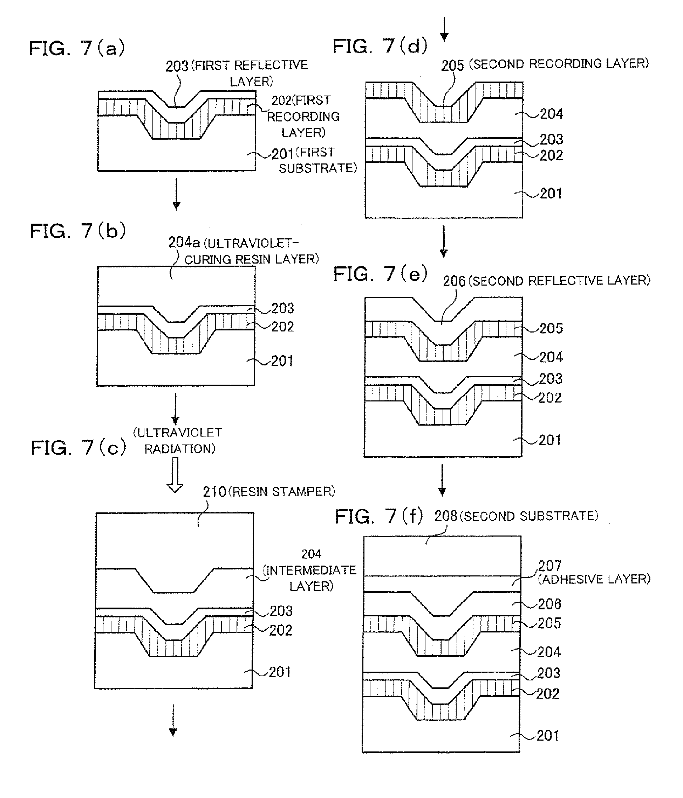

Figs. 7(a) to 7(f) illustrate a process of producing a dual layered optical recording medium according to the third embodiment of the present invention. As shown in Fig. 7(a), a first substrate 201 on the surface of which grooves and lands, and pre-pits are formed is first produced by injection molding using a stamper. Next, a first recording layer 202 is formed by applying a coating solution in which at least an organic dye is dissolved onto the patterned indented surface of the first substrate 201 by spin coating, and heating (annealing) to remove the solvent used for the coating solution. After forming the first recording layer 202, a semitransparent first reflective layer 203 is formed by sputtering on the first recording layer 202 by sputtering or depositing an Ag alloy.

As shown in Fig. 7(b), an ultraviolet-curing resin layer 204a is sequentially formed over the surface of the first reflective layer 203 by spin coating. As shown in Fig. 7(c), after application of the ultraviolet-curing resin layer 204a by spin coating, a resin stamper 210 is placed, and the pattern is transferred to the ultraviolet-curing resin layer 204a, while the thickness of the ultraviolet-curing resin layer 204a is adjusted to a predetermined range. In this state, the ultraviolet-curing resin layer 204a is cured by irradiating the side of the resin stamper 210 with ultraviolet, and after sufficient curing, the resin stamper 210 is released to form an intermediate layer 204 having the pattern on its surface.

The resin stamper 210 desirably has sufficient releasability from the resin to be the intermediate layer 204, excellent moldability, and high morphological stability. From the viewpoint of productivity and cost, the resin stamper 210 can be desirably used for several times of the transfer. Also, it can desirably be recycled after use. Examples of materials for the resin stamper 210 include acrylic resins, methacrylic resins, polycarbonate resins, polyolefin resins (in particular, amorphous polyolefins), polyester resins, polystyrene resins, and epoxy resins. Among these materials preferred are amorphous polyolefins from the viewpoint of high productivity such as moldability, cost, low hygroscopicity, and morphological stability.

As shown in Fig. 7(d), a second recording layer 205 is formed by applying a coating solution containing an organic dye to the surface of the intermediate layer 204 by spin coating, and heating (annealing) so as to remove the solvent used for the coating solution.

As shown in Fig. 7(e), a second reflective layer 206 is formed on the second recording layer 205 by sputtering and depositing an Ag alloy. As shown in Fig. 7(f), a mirror substrate as a second substrate 208 prepared by injection molding of polycarbonate is laminated to the second reflective layer 206 with an adhesive layer 207, to complete production of an optical recording medium.

The optical recording medium and the process of producing the medium according to the third embodiment of the present invention have been described, the embodiments of the present invention is not intended to limit to these embodiments, and can variously be changed. For example, the optical recording medium may have three or more recording layers. Other layers may be provided between each layer or as the outermost layer, if necessary. The medium can be applied to not only substrate-incidence type optical discs, but also coating-incidence optical discs that have a laminated structure composed of at least a substrate/a reflective layer/a second recording layer/a buffer layer/an intermediate layer/a semitransparent reflective coating/a first recording layer/a protective layer, and enables information to be recorded and read by irradiating the protective layer (that is, the coating) with laser beams.

A requirement to be suitable for dyes used for a multi-layered medium is higher recording sensitivity than that of dyes used for a single layer medium. This is because the phenomenon in which light absorption and transmission of light inevitably occur even on layers other than a layer for recording information during recording of the information on this layer. In this sense, the azacyanine dye of this application is more preferred due to superior recording sensitivity compared to that of the conventional one.

### [Examples]

The present invention will now be described in further detail with reference to Examples, but the present invention is not limited only to those Examples described below unless departing from the spirit of the present invention.

### [I. Preparation and Evaluation of Dye]

### [Example 1]

In 10 g of acetic acid, 2 g of 2-(1,3,3-trimethylindolin-2-ylidene)acetaldehyde and 2.43 g of 4-aminopentafluoroethylthiophenol were dispersed, and stirred for 6 hours at 80°C. The reaction mixture was spontaneously cooled, and poured into 200 ml of water, followed by addition of about 1 g of sodium iodide to precipitate crystal. The resultant crystal was filtered out. To the filtered crystal, 100 ml of acetone was added to dissolve the crystal. After insoluble matter was filtered off, the acetone was evaporated under reduced pressure. The resultant crystal was washed with diisopropyl ether (twice) and isopropyl alcohol (twice) to yield 2.93 g of yellow crystalline Exemplary Compound (1).

The resultant Exemplary Compound (1) had a maximum absorption wavelength (λₘₐₓ) of 427 nm, and a molar absorption coefficient of 5.2×10⁴ on measurement of absorbance in chloroform.

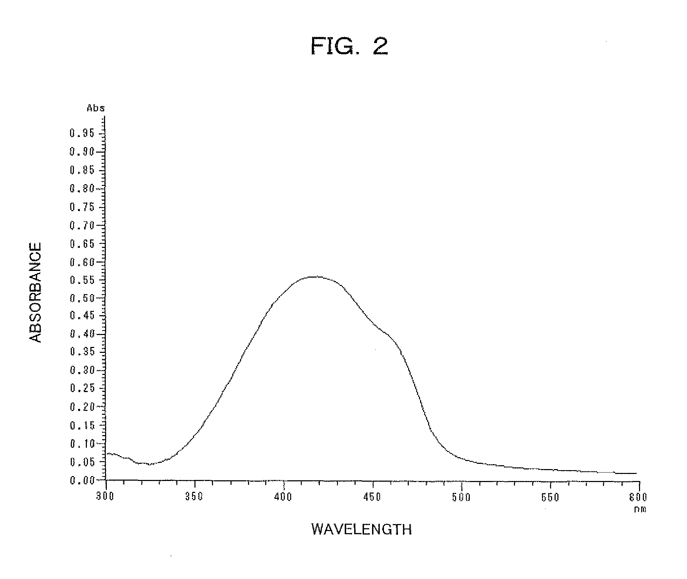

The resultant Exemplary Compound (1) was dissolved in octafluoropentanol into a concentration of 1 weight%, and insoluble matter was filtered off to prepared a dye solution. The resultant dye solution was placed dropwise on an injection-molded polycarbonate resin substrate with a diameter of 120 mm and a thickness of 1.2 mm, and applied by a spinning process (500 rpm), followed by drying at 100°C for 30 minutes to form a dye-coated film. This coated film had a maximum absorption wavelength (λₘₐₓ) of 419 nm on measurement of absorbance. Fig. 2 shows an absorption spectrum.

### [Example 2]

In 10 g of acetic acid, 2 g of 2-(1,3,3-trimethylindolin-2-ylidene)acetaldehyde and 1.37 g of 3,4-(methylenedioxy) aniline were dispersed, and stirred for 2 hours at 80°C. The reaction mixture was spontaneously cooled, and poured into 200 ml of water, followed by addition of about 1 g of sodium iodide to precipitate crystal. The resultant crystal is filtered out. The filtered crystal was dissolved in 100 ml of acetone, and insoluble matter was filtered off, followed by evaporation of acetone under reduced pressure. The resultant crystal was washed with 50 ml of diisopropyl ether to yield 1.32 g of Exemplary Compound (2).

The resultant Exemplary Compound (2) had a maximum absorption wavelength (λₘₐₓ) of 437 nm and a molar absorption coefficient of 3.9×10⁴ on measurement of absorbance in chloroform.

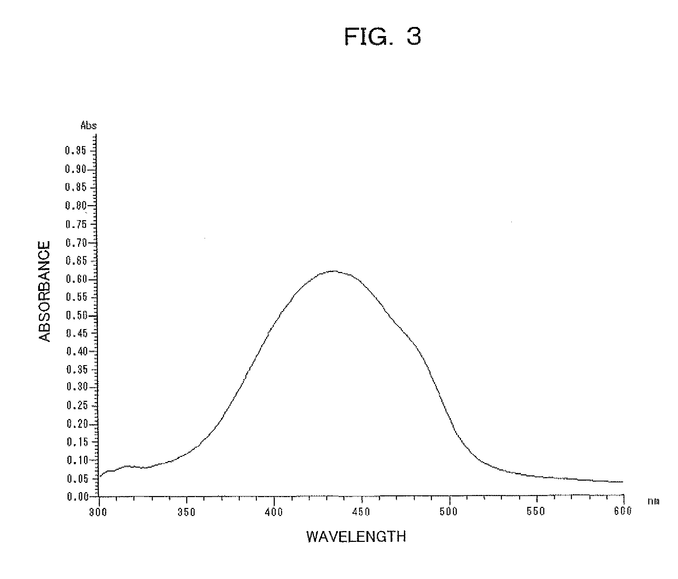

Using the resultant Exemplary Compound (2), a dye-coated film was formed on a polycarbonate resin substrate as in Example 1. This coated film had a maximum absorption wavelength (λₘₐₓ) of 435.5 nm on measurement of absorbance. Fig. 3 shows an absorption spectrum.

### [Example 3]

In 10 g of acetic acid, 2 g of 2-(1,3,3-trimethylindolin-2-ylidene)acetaldehyde and 1.40 g of 3-amino-5-tert-butylisoxazole were dispersed, and stirred for 5 hours at 50 to 60°C. The reaction mixture was allowed to cool, and poured into 100 ml of water, followed by addition of several grams of ammonium hexafluorophosphate (NH₄PF₆) to precipitate crystal. The resultant crystal was filtered out. The filtered crystal was stirred in 100 ml of water for 10 minutes, and then filtered out again. The resultant solid was dried to yield 2.79 g of yellow crystalline Exemplary Compound (15).

The resultant Exemplary Compound (15) had a maximum absorption wavelength (λₘₐₓ) of 395.5 nm and a molar absorption coefficient of 4.2×10⁴ on measurement of absorbance in chloroform.

### [Comparative Example 1]

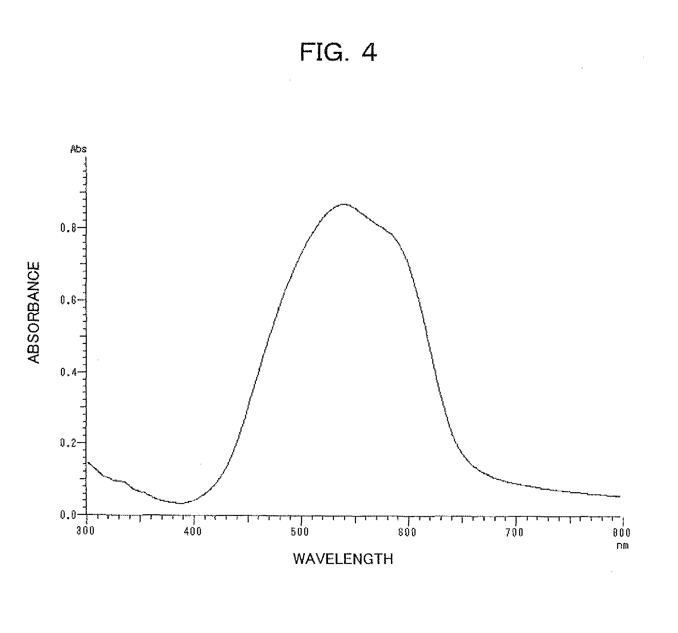

Using the dye represented by following structural formula (A), a dye-coated film was formed on the polycarbonate resin substrate by the procedure as in Example 1. This coated film was measured for absorbance. Fig. 4 shows the resultant absorption spectrum. The absorption peak was shown in a longer wavelength than 500 nm (539 nm), and the absorption in the vicinity of 400 nm was extremely low, which did not meet the purpose of the present invention using 405 nm laser.

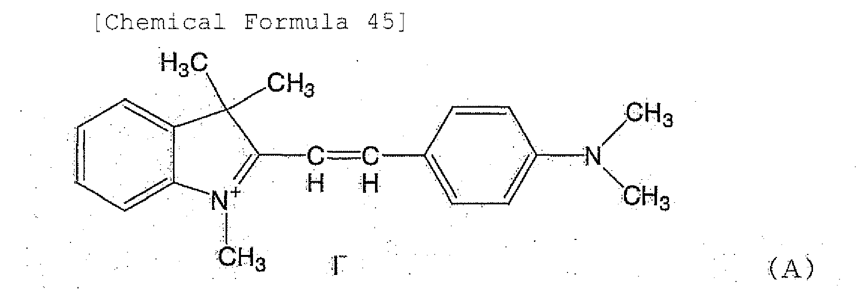

### [Comparative Example 2]

The dye represented by following structural formula (B) was applied to a polycarbonate substrate identical to that used in Example 1 under substantially the same conditions as those of Example 1. The coated film was crystallized and was not transparent, which did not meet the purpose of the present invention.

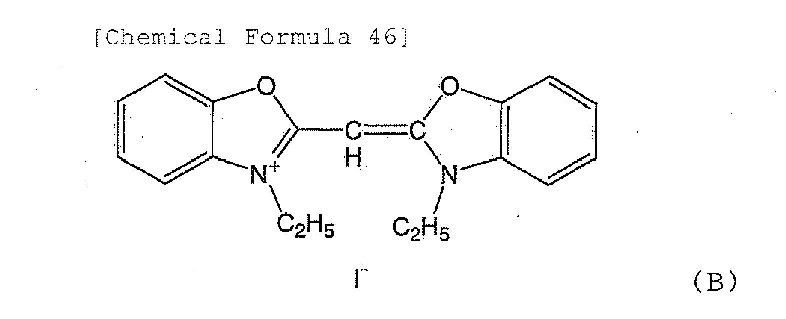

### [Examples 4 to 9]

Dye compounds corresponding to the azacyanine dye of formula [I] described above were produced by essentially the same procedure as that in Example 1, but with the change in the type of amine compound to react with 2-(1,3,3-trimethylindolin-2-ylidene)acetaldehyde (Examples 4 to 9).
Table 1 below shows the structure of the group R⁵ and the structure of the counter anion X⁻ in formula [I] for the dye compounds prepared in Examples 1 to 9. In Table 1, "Pr(i)" means i-propyl group, and "t-Bu" means t- butyl group. For any dye compound prepared in Examples 1 to 9, the groups R¹ to R³ in above-described formula [I] means methyl group, and the group R⁴ means hydrogen atom. In Table 1, each of the dye compounds prepared in Examples 1 to 9 is assigned a number which corresponds to Exemplary Compounds (1) to (19).

Each of the dye compounds prepared in Examples 4 to 9 was also measured for maximum absorption wavelength (λₘₐₓ) in chloroform, maximum absorption wavelength (λₘₐₓ) of the coated film, and molar absorption coefficient in chloroform by the procedure as in Example 1.

### [Evaluation]

For the dye compounds prepared in Examples 1 to 9, Table 1 below shows maximum absorption wavelength in chloroform (the column of "λ sol" in Table 1), maximum absorption wavelength of the coated film (the column of "λ film" in Table 1), and molar absorption coefficient in chloroform (the column of "ε" in Table 1).

[Table 1]

**Table 1**

| Example | Exemplary Compound | R⁵ | X⁻ | λ sol (nm) | λ film (nm) | ε (× 10⁴) |
|---|---|---|---|---|---|---|
| 1 | (1) | 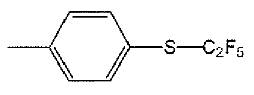 | I⁻ | 419 | 427 | 6.1 |
| 2 | (2) | 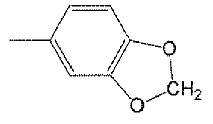 | Cl⁻ | 437 | 433,5 | 3.9 |
| 3 | (15) | 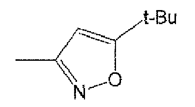 | PF₆⁻ | 395.5 | 389.5 | 4.2 |
| 4 | (6) | 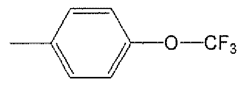 | I⁻ | 422 | 416 | 4.5 |
| 5 | (20) | 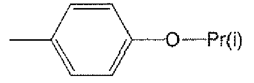 | I⁻ | 434.5 | 425 | 4.4 |
| 6 | (3) | 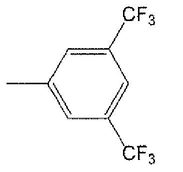 | I⁻ | 425.5 | 424 | 4.6 |
| 7 | (21) | 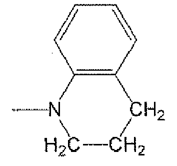 | I⁻ | 419.5 | 424 | 4.4 |
| 8 | (22) | 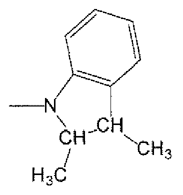 | PF₆⁻ | 425 | 422.5 | 5.8 |
| 9 | (23) | 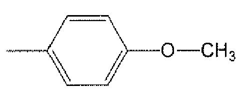 | Cl⁻ | 397.5 | 493 | 2.8 |

### [II. Preparation and Evaluation of Optical Recording Medium]

On the dye-coated film produced in Example 1 and 2, a reflective layer is formed by depositing Ag by sputtering, if necessary, and a protective layer is further formed by applying a UV-curing resin by spin coating and curing the resin by irradiation with UV, which forms an optical recording medium. This optical recording medium enables recording and reading, for example, by semiconductor laser beams with a center wavelength of 405 nm, considering the maximum absorption wavelength (λₘₐₓ) of the dye-coated film. That is, Exemplary Compounds (1) and (2) prepared in Examples 1 and 2 (the azacyanine dyes of the present invention) have a structure effective for blue laser beam recording.

Using Exemplary Compound (2) prepared in Example 2, an optical recording medium was produced according to the following procedure.
A dye solution was prepared by dissolving Exemplary Compound (2) prepared in Example 2 in octafluoropentanol into a concentration of 1.4 weight%, and filtering off insoluble matter. The dissolved solution was placed dropwise on an injection-molded polycarbonate resin substrate with a diameter of 120 mm and a thickness of 0.6 mm having grooves with a track pitch of 400 nm, a groove width of 200 nm, and a groove depth of 70 nm, and applied by a spinning process. During the coating, the number of revolutions was increased from 600 rpm to 4900 rpm over 25 seconds, and then maintained at 4900 rpm for 5 seconds. Then, by drying at 100°C for 30 minutes, a recording layer was formed. On this recording layer, a silver alloy was deposited into a film with a thickness of 100 nm by sputtering, which gave a reflective layer. A protective coating agent composed of a UV-curing resin was then applied by a spinning process, and irradiated with UV beams to form a protective layer with a thickness of 5 µm. A polycarbonate substrate with a thickness of 0.6 mm was bonded onto the face of the protective layer with a delayed-curing adhesive to prepare an optical recording medium for evaluation.

While spinning the resultant optical recording media for evaluation at a linear velocity of 6.61 m/sec, single-frequency signals of 8T mark/8T space were recorded on the grooves by laser beams with a wavelength of 405 nm (the numerical aperture of the object lens NA=0.65), where T means a reference clock cycle corresponding to a frequency of 65 MHz. The following recording pulse strategy was used: the division pulse number (n-1) with a mark length nT, a first recording pulse width of 2T, a subsequent recording pulse width of 0.6T, a bias power of 1.5 mW, a reading power of 0.4 mW, and a variable recording power. As a result, a signal with a modulation rate of 51% was recorded at 7 mW. The modulation rate is believed to increase further by optimization of recording conditions such as the pulse strategy.

The description above shows that use of the dye of the present invention enables information to be recorded by short wavelength laser. In addition to this, to confirm the dye of the present invention to satisfy more astringent conditions, that is, characteristics required for dyes used for high-density optical discs, the dye was evaluated according to the HD DVD-R Specification.

### [Example A]

Onto a polycarbonate substrate with a thickness of 0.6 mm, a track pitch of 0.4 µm, a groove width of 260 nm, and a groove depth of 60 nm, a solution prepared by dissolving Exemplary Compound (27) in TFP (2, 2, 3, 3-tetrafluoro-1-propanol) into a concentration of 1.0 weight% was applied by spin coating, and dried at 70°C for 25 minutes to provide a recording layer. Its absorbance at 470 nm was 0.32 as measured using air as a reference. On this recording layer, an AgBi_{0.2}Nd_{0.5} reflective film with a thickness of 120 nm was then provided by sputtering. Onto the reflective layer, a polycarbonate backing plate having a thickness of 0.6 mm was bonded with an ultraviolet-curing resin (SK7100 from Sony Chemicals Corporation) to produce an optical recording medium (an optical recording medium of Example A).

Recording was conducted on the resultant optical recording medium of Example A, using a tester with a laser wavelength of 405 nm and an NA (numerical aperture) of 0.65 (ODU-1000 from PULSTEC) at a linear velocity of 6.61 m/s and a shortest mark length of 204 nm. The recording and reading were evaluated according to HD DVD-R Specification ver. 1.0 defined by the DVD forum for the PRSNR (Partial Response SNR) in the specification.

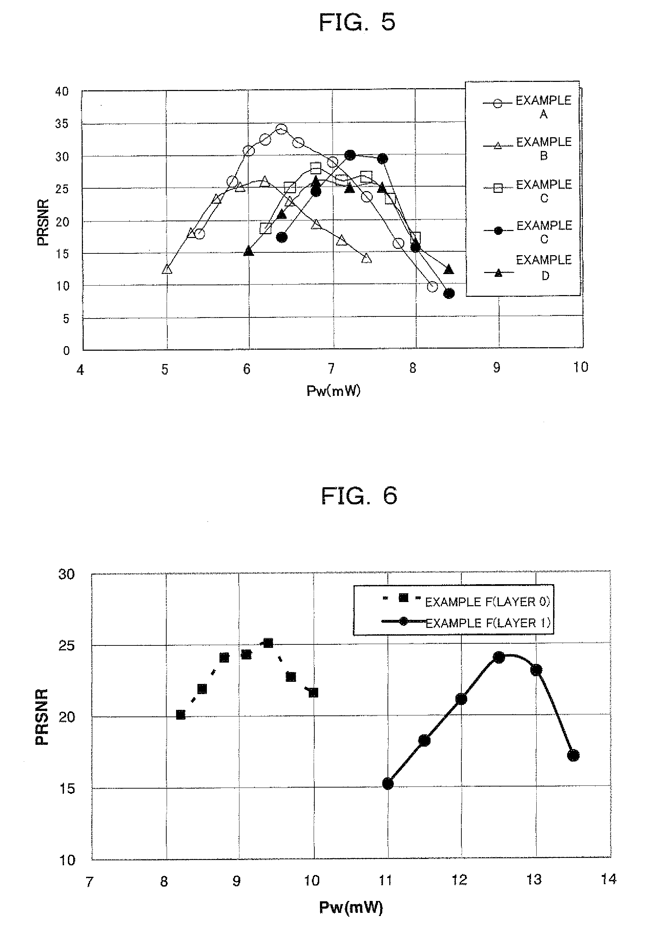

The result shows that the recording mechanism was a Low-to-High type, and that the optimum recording power was 6.4 mW. Fig. 5 shows the results of the PRSNR. As shown from Fig. 5, the PRSNR for the optical recording medium of Example A was 34.0, which is much higher than 15 being the criterion measure.

### [Example B]

An optical recording medium (an optical recording medium of Example B) was produced as in Example A except that Exemplary Compound (28) was used as a dye, and evaluated under the same conditions.

The result shows that the recording mechanism was a Low-to-High type, and that optimum recording power was 6.2 mW. Fig. 5 shows the results of the PRSNR. As shown from Fig. 5, the PRSNR for the optical recording medium of Example B was 26.0, which is much higher than 15 being the criterion measure.

### [Example C]

An optical recording medium (an optical recording medium of Example C) was produced as in Example A except that Exemplary Compound (29) was used as a dye, and evaluated under the same conditions.

The result shows that the recording mechanism was a Low-to-High type, and that optimum recording power was 6.8 mW.
Fig. 5 shows the results of the PRSNR. As shown from Fig. 5, the PRSNR for the optical recording medium of Example C was 28.0, which is much higher than 15 being the criterion measure.

### [Example D]

An optical recording medium (an optical recording medium of Example D) was produced as in Example A except that Exemplary Compound (30) was used as a dye, and evaluated under the same conditions.

The result shows that the recording mechanism was a Low-to-High type, and that optimum recording power was 7.2 mW. Fig. 5 shows the results of the PRSNR. As shown from Fig. 5, the PRSNR for the optical recording medium of Example D was 30.0, which is much higher than 15 being the criterion measure.

### [Example E]

An optical recording medium (an optical recording medium of Example E) was produced as in Example A except that Exemplary Compound (31) was used as a dye, and evaluated under the same conditions.

The result shows that the recording mechanism was a Low-to-High type, and that optimum recording power was 6.8 mW. Fig. 5 shows the results of the PRSNR. As shown from Fig. 5, the PRSNR for the optical recording medium of Example E was 26.0, which is much higher than 15 being the criterion measure.

### [Example F]

The same dye as that in Example A, that is, Exemplary Compound (27) was used to produce a dual layered medium, and the recording layers (Layer 0 and Layer 1) were evaluated for recording characteristics. Layer 0 refers to "first recording layer" (a recording layer adjacent the laser incidence), and Layer 1 refers to "second recording layer" (a recording layer distant from the laser incidence). Also, an AgBi_{1.0} alloy was deposited into a film with a thickness of 20 nm as a "first reflective layer" (a semitransparent reflective layer), Ag was deposited into a film with a thickness of 120 nm as a "second reflective layer", and an ultraviolet-curing resin was applied into a film with a thickness of 25 µm as an intermediate layer. The substrate used had a track pitch of 0.4 µm, a groove depth of 60 nm for Layer 0 and 65 nm for Layer 1, and a groove width for both Layers of 260 nm, and the absorbances at 470 nm were 0.315 for Layer 0 and 0.345 for Layer 1.

Under the same conditions as those for the single layer medium, recording, and particularly random pattern recording was conducted using a tester with a laser wavelength of 405 nm and an NA (numerical aperture) of 0.65 (ODU-1000 from PULSTEC) at a linear velocity of 6.61 m/s and a shortest mark length of 204 nm. The recording and reading were evaluated according to HD DVD-R Specification ver. 1.0 defined by the DVD forum for the PRSNR (Partial Response SNR) in the specification.

The evaluation results show that the recording mechanism was Low-to-High for both Layer 0 and Layer 1. Fig. 6 shows the results of the PRSNR. As shown in Fig. 6, Layer 0 had an optimum recording power of 9.4 mW and PRNSR of 25.1, and Layer 1 had an optimum recording power of 12.5 mW and PRNSR of 24.0. Both of the PRSNR for Layer 0 and Layer 1 were excellent that are much higher than 15.0 being the criterion measure. This can ensure recording characteristics essential for a dual layered medium.

### Industrial Applicability

The present invention is applicable to any field, and preferably to the field of optical recording media in which recording and reading are conducted by a laser beam with a short oscillation wavelength, such as a blue laser beam.

Although the present invention has been described in detail with reference to particular embodiments, it is apparent to those skilled in the art that various modifications and variations can be made thereto without departing from the spirit and scope of the present invention.
The present application is based on Japanese Patent Application (Patent Application No. 2005-375871) filed on December 27, 2005, and Japanese Patent Application (Patent Application No. 2006-174427) filed on June 23, 2006, which are herein incorporated in their entireties by reference.

## Claims

1. An optical recording medium comprising:
at least a substrate, and a recording layer thereon that can record or read information by irradiation with light,
the recording layer containing an azacyanine dye represented by following general formula [I]: 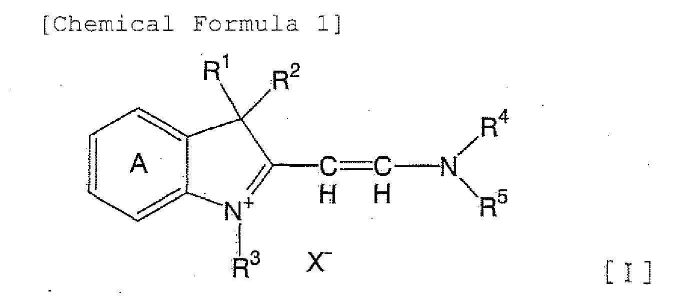
wherein
R¹ and R² each independently represent a hydrogen atom or an optionally substituted linear or branched alkyl group having one to four carbon atoms; or R¹ and R² may also be combined together to form a ring;
R³ represents a hydrogen atom or a hydrocarbon group, and R³ may crosslink with two or more compounds of general formula [I] when R³ represents a hydrocarbon group;
R⁴ represents a hydrogen atom or a linear or branched alkyl group having one to four carbon atoms; and
R⁵ represents an optionally substituted aromatic ring group or an optionally substituted unsaturated heterocyclic group, and R⁵ may combine with two or more compounds of general formula [I];
provided that R⁴ and R⁵ may be combined together to form a ring;
X⁻ represents a counter anion; and
the benzene ring A may be optionally substituted.

2. The optical recording medium according to claim 1, wherein in general formula [I], R⁴ represents a hydrogen atom, and R⁵ represents an optionally substituted phenyl group.

3. The optical recording medium according to claim 1, wherein in general formula [I], R⁴ represents a hydrogen atom, and R⁵ represents an optionally substituted five- or six-membered unsaturated heterocyclic group.

4. The optical recording medium according to claim 1, wherein R⁴ and R⁵ in general formula [I] are combined to form a five- or six-membered saturated hydrocarbon group or saturated heterocycle.

5. The optical recording medium according to claim 2, wherein R⁵ represents an unsubstituted phenyl group.

6. The optical recording medium according to any one of claims 1 to 5, wherein X⁻ in general formula [I] is an azo-metal complex anion represented by following general formula [II]: 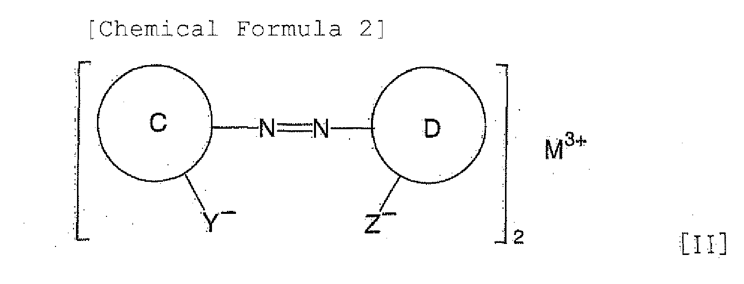 wherein
these rings C and D each independently represent an aromatic ring or a heterocycle provided that at least one of these rings C and D is a heterocycle;
and Z each independently represent a group having active hydrogen; and
M represents a trivalent metal element.

7. The optical recording medium according to any one of claims 1 to 6, wherein on the recording layer, recording or reading of information is conducted using a laser beam with a wavelength of 350 nm to 530 nm.

8. An azacyanine dye represented by following formula (i): 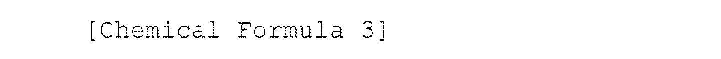 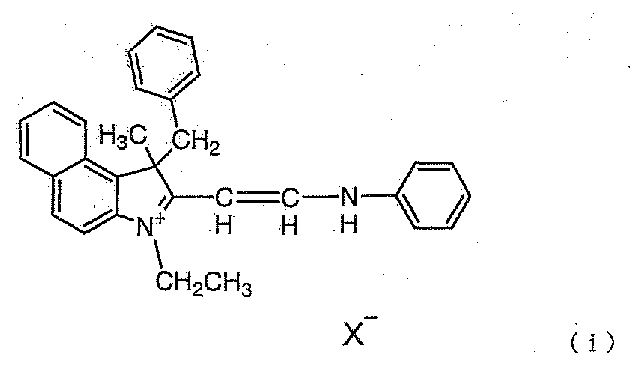 wherein X⁻ represents a counter anion.
